(19) Europäisches Patentamt
European Patent Office
Office européen des brevets

(11) Publication number: **0 015 065**
**B1**

(12) # EUROPEAN PATENT SPECIFICATION

(45) Date of publication of patent specification: **28.12.83**

(21) Application number: **80300216.1**

(22) Date of filing: **23.01.80**

(51) Int. Cl.³: **C 07 D 487/04,**
A 61 K 31/505 //C07C79/46,
C07C101/52, C07C109/10,
C07C149/40, C07C149/43,
C07D265/26, C07D333/24,
C07D413/04, (C07D487/04,
239/00, 231/00)

(54) **Pyrazolo (5,1-b) quinazolin-9(4H)-one derivatives, process for their preparation and pharmaceutical compositions containing them.**

(30) Priority: **24.01.79 US 6044**
**24.01.79 US 6045**
**24.01.79 US 6046**
**11.01.80 US 111148**
**11.01.80 US 111160**
**11.01.80 US 111147**

(43) Date of publication of application:
**03.09.80 Bulletin 80/18**

(45) Publication of the grant of the patent
**28.12.83 Bulletin 83/52**

(84) Designated Contracting States:
**BE CH DE FR GB IT NL SE**

(56) References cited:
**DE - A - 1 111 505**
**FR - A - 2 335 229**
**US - A - 4 105 766**

(73) Proprietor: **WARNER-LAMBERT COMPANY**
**201 Tabor Road**
**Morris Plains New Jersey 07950 (US)**

(72) Inventor: **Sircar, Jagadish Chandra**
**3615 Charter Place**
**Ann Arbor, Michigan 48105 (US)**
Inventor: **Capiris, Thomas**
**14816 Greenbriar Court**
**Plymouth, Michigan 48170 (US)**
Inventor: **Kesten, Stephen Jacob**
**3181 Oak Drive**
**Ypsilanti, Michigan 48197 (US)**

(74) Representative: **Jones, Michael Raymond et al,**
**HASELTINE LAKE & CO. 28 Southampton**
**Buildings Chancery Lane**
**London WC2A 1AT (GB)**

**EP 0 015 065 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Pyrazolo [5,1-b] quinazolin -9(4H)-one derivatives process for their preparation and pharmaceutical compositions containing them

This invention relates to pyrazolo [5,1-b] quinazolin -9(4H)-one derivatives useful as anti-allergy agents, processes for their production and pharmaceutical compositions containing them.

Certain pyrazoloquinazoline carboxylic acids are known. Thus, for instance, United States Patents Nos. 3,150,136 and 3,167,537 disclose, *inter alia,* certain pyrazoloquinazoline carboxylic acids which are useful as intermediates for the preparation of dyestuffs. Also, German Patent No. 1,111,505 discloses substituted 2-carboxy-pyrazolo-[5,1-b] quinazolin-8-(4H)-ones which are useful as photographic colour developers. However, none of these three references discloses or suggests any pharmaceutical utility for these acids, nor do they disclose the corresponding tetrazole or carboxamidotetrazole derivatives.

United States Patent No. 4,105,766 (assigned to Sterling Drug Inc.) discloses 4,5-dihydro-5-oxypyrazolo [1,5-a] quinazoline-3-carboxylic acids and lower alkyl esters and alkali metal salts thereof having the formula:

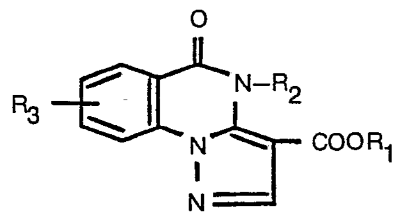

wherein $R_1$, $R_2$ and $R_3$ are selected atoms or radicals. Certain pharmaceutical activity is claimed for these compounds, including anti-allergic activity for some of the compounds, anti-inflammatory activity for other of the compounds, and anti-parasitic activity for yet a third group of the compounds. The table near the foot of column 10 of United States Patent No. 4105766 shows the results obtained during anti-allergy tests, from which it can be seen that of the numerous compounds tested in accordance with a standard test only two compounds gave a % inhibition greater than 75%; in fact the two compounds give inhibitions of 81% and 92% but this was when the standard dose, p.o., was as high as 100 mg/kg, and even the control (known in the art) used in the tests gave a % inhibition of 62% under the same standard conditions. Compounds administered p.o. have an activity which is less than when administered intraperitoneally or intravenously.

French Patent Specification No. 2335229 (C. H. Boehringer Sohn) discloses various quinazolone derivatives having the formula:

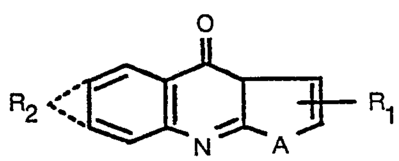

where $R_1$ and $R_2$ are selected substituents and A is —CH=CH—, —CH=N— or —S—. Thus the right hand ring can be a 5-membered ring but only when A represents —S—. If the right hand ring is to contain 2 nitrogen atoms, then A must represent —CH=N—, in which case the right hand ring is a six membered ring with the two nitrogen atoms *not* immediately adjacent to each other. The compounds of French Patent Specification No. 2335229 have anti-allergy activity, and certain of those compounds were treated using a standard test described on page 5 of French Patent Specification No. 2335229, involving the inhibition of passive cutaneous anaphylaxis (PCA). With five of the six compounds tested, the % inhibition ranged from 48 to 88% when using a dose as high as 100 mg/kg. In only one instance was 100% inhibition achieved, and that required a dose as high as 30 mg/kg.

According to the present invention there is provided a compound having the following general formula:—

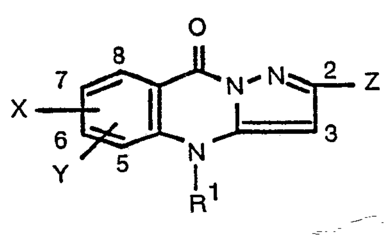

(I)

wherein X is hydrogen, hydroxy, alkyl having from 1 to 6 carbon atoms, alkoxy having from 1 to 6 carbon atoms, halogen trifluoromethyl, or $SO_nR$ wherein R is alkyl having from 1 to 6 carbon atoms and

n is 0, 1 or 2; Y is hydrogen, hydroxy, alkyl having from 1 to 6 carbon atoms, alkoxy having from 1 to 6 carbon atoms, or 2-tetrahydrothienyl; $R^1$ is hydrogen or alkyl having from 1 to 6 carbon atoms; Z is COOH,

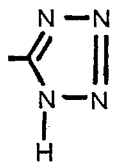

or

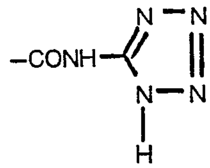

or a pharmaceutically acceptable salt thereof; provided that when all of X, Y and $R^1$ are hydrogen, Z is not COOH.

Preferred compounds are those having the following formula I:

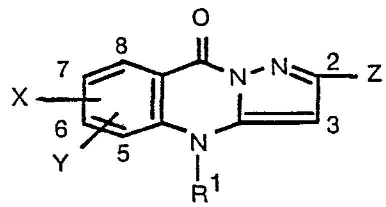

(I)

wherein X is hydrogen, hydroxy, methyl, methoxy, chlorine, fluorine or another halogen, or trifluoromethyl; Y is hydrogen or methoxy; Z is —COOH,

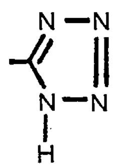

or

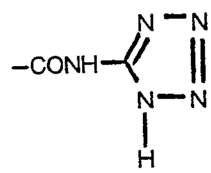

and $R^1$ is hydrogen, methyl or ethyl; with the proviso that, when $R^1$ is hydrogen and Z is

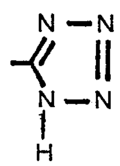

,

X is hydrogen, methyl, methoxy, chlorine, fluorine, trifluoromethyl or hydroxy and Y is hydrogen or, when X is methoxy, is methoxy; or with the proviso that, when $R^1$ is methyl or ethyl and Z is

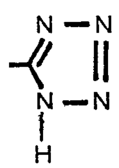

,

Y is hydrogen and X is hydrogen, methyl, methoxy or halogen; or with the proviso that, when Z is

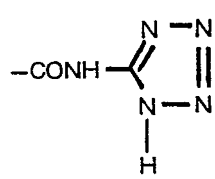

,

$R^1$ is hydrogen or methyl, X is hydrogen, methyl, methoxy, hydroxy, or chlorine, and Y is hydrogen or, when $R^1$ is methoxy, is methoxy or, with the proviso that, when all of X, Y and $R^1$ are hydrogen, Z is not COOH: or a pharmaceutically acceptable salt thereof.

The present invention also provides a pharmaceutical composition comprising an anti-allergic effective amount of a compound of the formula I:

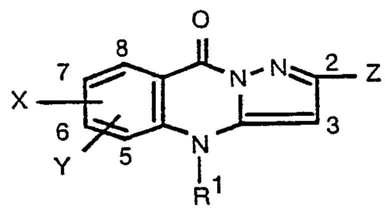

(I)

wherein X is hydrogen, hydroxy, alkyl having from 1 to 6 carbon atoms, alkoxy having from 1 to 6 carbon atoms, halogen, trifluoromethyl, or $SO_nR$ wherein R is alkyl having from 1 to 6 carbon atoms, and n is 0,1 or 2; Y is hydrogen, hydroxy, alkyl having from 1 to 6 carbon atoms, alkoxy having from 1 to 6 carbon atoms, or 2-tetrahydrothienyl; $R^1$ is hydrogen or alkyl having from 1 to 6 carbon atoms; Z is COOH,

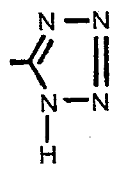

or

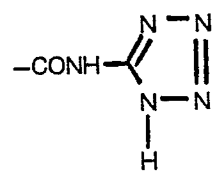

with the proviso that, when all of X, Y and $R^1$ are hydrogen, Z is not COOH, or a pharmaceutically acceptable salt thereof: and a diluent or carrier for that compound or salt thereof.

The compounds of the present invention are suitable for use in preventing the allergic response in a mammal, by administering to said mammal an anti-allergic effective amount of a compound of formula I or a pharmaceutically acceptable salt thereof.

In the production of some of the compounds of the present invention there can be used a compound of the formula II:

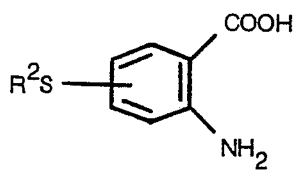

II

wherein $R^2$ is alkyl of from 1 to 6 carbon atoms.

The tetrazoles of the present invention, that is compounds of the formula I wherein Z is

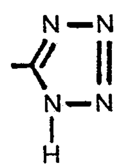

i.e.

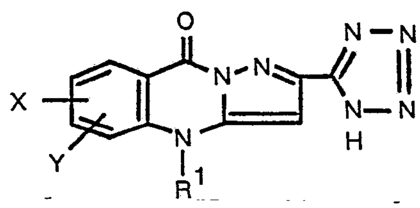

I

and X, Y and $R^1$ are as defined above may be prepared from the corresponding acids or esters by methods familiar to those skilled in the art. For example the properly substituted carboxylic acid may be converted to the corresponding acid halide such as the chloride by treatment with thionyl chloride or oxalyl chloride and converted to the acid amide by treatment with ammonia. The amide is dehydrated by treatment with, for example phosphorus oxychloride or thionyl chloride in dimethylformamide thereby producing the corresponding nitrile which when treated with sodium azide and ammonium chloride, for example, will yield the corresponding tetrazole. The above-described amides may also be prepared directly from the corresponding esters by treatment with, for example, gaseous ammonia by methods familiar to those skilled in the art.

Other methods and reagents for converting carboxylic acids or esters into the corresponding tetrazoles will be familiar to those skilled in the art.

The carboxyamido tetrazoles of the invention, i.e. compounds of the formula I wherein Z is

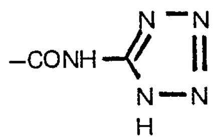

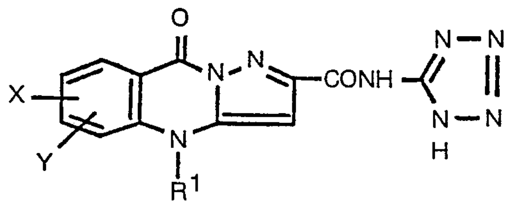

and X, Y and R¹ are as defined above may be prepared from the corresponding carboxylic acids by methods familar to those skilled in the art. For example, the properly substituted carboxylic acid may be converted to the corresponding acid halide such as the chloride by treatment with thionyl chloride or oxalyl chloride. Treatment of the halide with 5-aminotetrazole produces the carboxamido tetrazoles of formula I. Alternatively, a carboxylic acid may be treated with 1,1-carbonyldiimidazole to produce a compound of formula III.

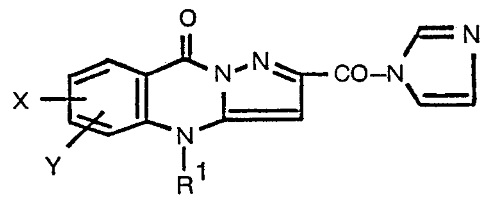

III.

Treating the so produced compound of formula III with 5-aminotetrazole, for example in dimethylformamide solution, will produce the corresponding carboxamidotetrazole of formula I. Those skilled in the art will recognize that the intermediate carbonylimidazole of formula III need not be isolated prior to being treated with 5-aminotetrazole. In addition, the properly substituted carboxylic acid may be directly coupled with 5-aminotetrazole by use of such agents as N-ethoxycarbonyl-2-ethoxy-1,2-dihydroquinoline (EEDQ), or dicyclohexylcarbodiimide (DCC).

The above-described 2-carboxy-4-alkyl-pyrazolo-[5,1-b]quinazolin-9(4H)-ones, i.e., compounds of formula I wherein Z is COOH and R¹ is alkyl of from 1 to 6 carbon atoms may be prepared by alternate procedures, which are considered equivalent for purposes of the invention. In one such procedure a 3-alkoxycarbonyl-2-pyrazolin-5-one having formula IV

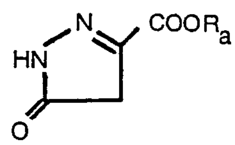

IV

wherein $R_a$ is any convenient alkyl group, preferably of from 1 to 6 carbon atoms, most preferably ethyl, is reacted in the presence of a strong base such as sodium hydride with a substituted N-alkyl isatoic anhydride of formula V

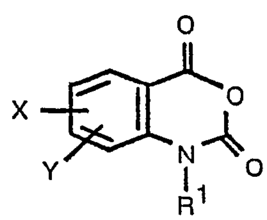

V,

producing a 4-alkyl-4,9-dihydro-9-oxo-pyrazolo[5,1-b]-quinazoline-2-carboxylic acid ester having formula VI

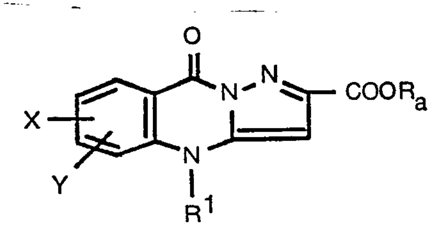

VI

wherein X, Y, R¹ and $R_a$ are as described above. The ester function of the compound of formula VI may be hydrolyzed by any convenient procedure to produce the corresponding acid, i.e., the compound of formula I wherein Z is COOH.

The 3-alkoxycarbonyl-2-pyrazolin-5-ones of formula IV may be prepared by the procedure of R. V. Rothenberg, J. Prakt. Chem., *2* 53 (1895).

The substituted N-alkyl isatoic anhydrides of formula V may be prepared by the procedure of G. E. Hardtmann, et al., J. Hetero. Chem., *12*, 565 (1975); these compounds may also be prepared by alkylating the corresponding N-unsubstituted isatoic anhydride with themselves may be prepared for example, by reacting a properly substituted anthranilic with phosgene, as described below. Several of these compounds are also commercially available from the Aldrich Chemical Company, Milwaukee, Wisconsin and the Sherwin Williams Company, Cleveland, Ohio.

The above-described 2-carboxypyrazolo[5,1-b]-quinazolin-9(4H)-ones, i.e., compounds of formula I wherein Z is COOH and $R^1$ is hydrogen may be prepared by alternate procedures, which are considered equivalent for purposes of the invention. One such procedure involves the reaction of a substituted anthranilic acid of the formula

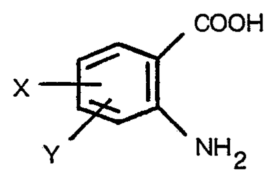

wherein X and Y are as defined above with phosgene to produce the corresponding N-unsubstituted isatoic anhydride of the formula

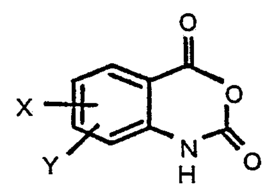

This reaction is conveniently carried out by adding, with cooling, a solution of phosgene in benzene to a solution of the anthranilic acid in, for example, dioxane/benzene (3:1). The isatoic anhydride is then converted to a 2-aminobenzoic acid hydrazide of the formula

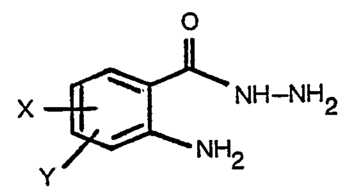

by treatment with, for example, an aqueous hydrazine hydrate solution. The hydrazide is next converted to the desired 2-carboxypyrazolo[5,1-b]quinazolin-9(4H)-ones by treatment with a dialkyl oxalacetate, preferably diethyl oxalacetate sodium salt, for example, in aqueous solution, followed by hydrolysis of the ester to the carboxylic acid.

The thioalkyl anthranilic acids of formula II which are utilized to prepare the corresponding thioalkyl substituted isatoic anhydrides are novel, and may themselves be prepared by alternate procedures which are considered equivalent for purposes of the invention. One such procedure involves the steps of treating a halo-substituted 2-nitrobenzoic acid with sodium sulfide; alkylating the so produced mercaptan; followed by reduction of the nitro group thereby producing the desired thioalkyl substituted anthranilic acid II. The above-described alkylated mercaptan may also be produced by treating the halo-substituted 2-nitrobenzoic acid with a mercaptide such as a sodium mercaptide. The starting halo-substituted 2-nitro-benzoic acids are either commercially available or may be prepared by methods known to those skilled in the art. For example, 5-chloro-2-nitrobenzoic acid is available from Aldrich Chemical Company, Milwaukee, Wisconsin 53233. For purposes of the invention, the preferred thioalkyl anthranilic acids are represented by the following formula:

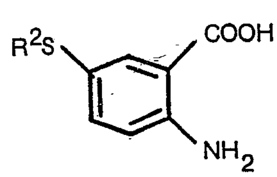

J. Pharm. Soc. Japan, *72* 76 (1952), [C.A.: *46*, 11150h (1952)] discloses ethyl 5-thioethylanthranilate.

In an alternate procedure to prepare the N-alkylated compounds of the formula I, a compound of the formula VII

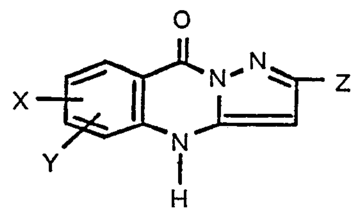

VII

wherein X, Y and Z are as defined above may be N-alkylated by standard procedures to produce the corresponding N-alkyl derivative.

The compounds of the invention of formula I are acids or are acidic in nature and form pharmaceutically acceptable salts with both organic and inorganic bases such as dimethylaminoethanol, the alkali metal and alkaline earth hydroxides and the alkali metal carbonates and bicarbonates such as lithium, sodium, potassium and calcium hydroxide, and the carbonates and bicarbonates of lithium, sodium and potassium. The salts are prepared by reacting an acid or the tetrazole with the desired base in the conventional manner. The tetrazoles and acids differ from their respective salts somewhat in certain physical properties such as solubility in polar solvents, but the salts are otherwise equivalent to their respective tetrazoles or acids for purposes of the invention.

The compounds of the invention can exist in unsolvated as well as solvated forms, including hydrated forms. In general, the solvated forms, with pharmaceutically acceptable solvents such as water or ethanol are equivalent to the unsolvated forms for purposes of the invention.

The thioalkyl groups, alkoxy groups and alkyl groups contemplated by the invention comprise both straight and branched carbon chains of from 1 to 6 carbon atoms. Representative of such groups are methyl, ethyl, isopropyl, pentyl, 3-methylpentyl, methoxy, ethoxy, propoxy, 1-ethylbutoxy, pentoxy, methylthio, isopropylthio and *n*-butylthio. The term halo is intended to include fluorine, chlorine, bromine and iodine.

The compounds of the invention of formula I are new chemical substances of value as pharmacological agents which prevent the allergic response in mammals by inhibition of the release of such allergic mediators, as histamine. The assay by which this utility was established is carried out as follows.

Rat Reaginic Passive Cutaneous Anaphylaxis (PCA).

The PCA test (D. J. Herzig, P. R. Schumann, E. J. Kusner, L. Robichaud, R. E. Giles, B. Dubnick, M. von Strandtmann, S. Klutchko, M. Cohen, and J. Shavel, Jr., "Immunopharmacology", M. E. Rosenthale and H. C. Mansmann, Eds., Spectrum Publications, Inc., New York, N.Y., 1975, pp. 103—124) involved immunization of rats with 1 mg of ovalbumin intramuscularly and approximately $10^{10}$ *B. pertussis* organisms as pertussis vaccine, intraperitoneally. Fourteen days later, the rats were bled and the serum was prepared. Suitable dilutions of antiserum were injected intradermally at various sites on the back of rats 48 hrs before an intravenous injection of 1 mg of ovalbumin in 1 ml of physiological saline and 0.25% Evans Blue. Thirty minutes later the animals were killed in ether, the dorsal skin was reflected, and the mean orthogonal diameter of the wheal was measured. For oral or intraperitoneal dosing, the drugs were suspended in 1% gum tragacanth in physiological saline and given 10—15 min before intravenous antigen challenge. For intravenous dosing, the compounds were dissolved in the saline/ovalbumin/Evans Blue solution and given with the antigen. If necessary, the compounds were first dissolved in a slight molar excess of sodium bicarbonate and then diluted into the antigen solution. Groups of five animals were used for all dose levels and control groups.

To quantitate the PCA test, the mean diameter of each wheal spot was graphed as a function of the relative anti-serum concentration. The line, fitted by the least-squares equation, was extrapolated to the value at "zero" antiserum concentration (base value). The following equation was then used to calculate the percent inhibition:

$$\%\ \text{inhibition} = [1- (\frac{\text{diameter of drug—base value}}{\text{diameter of control—base value}})] \times 100$$

The statistical significance of the results was determined by Student's *t* test ($p \leq 0.05$). An inhibition of 15% was significant.

Test results obtained for several preferred compounds of the invention are as follows: 7-methoxy-2(1H-tetrazol-5-yl)-pyrazolo[5,1-b]quinazolin-9-(4H)-one shows a 100% inhibition of allergic response when administered intraperitoneally to the rat at a dose of 5 mg/kg; 4,9-dihydro-6,7-dimethoxy-9-oxopyrazolo[5,1-b]quinazoline-2-carboxylic acid shows a 100% inhibition of the allergic response when administered intravenously to the rat at a dose of 0.1 mg/kg; 4,9-dihydro-7-fluoro-9-

oxopyrazolo[5,1-b]-quinazoline-2-carboxylic acid shows a 62% inhibition of the allergic response when administered intravenously to the rat at a dose of 0.1 mg/kg; 4,9-dihydro-7-(methylsulfinyl)-9-oxo-pyrazolo-[5,1-b]quinazoline-2-carboxylic acid shows a 76% inhibition of the allergic response when administered intraperitoneally to the rat at a dose of 5 mg/kg; 4,9-dihydro-7-(methylsulfonyl)9-oxo-pyrazolo[5,1-b]-quinazoline-2-carboxylic acid shows a 100% inhibition of the allergic response when administered intraperitoneally to the rat at a dose of 5 mg/kg; 4-methyl-7-methoxy-2-(1H-tetrazol-5-yl)-pyrazolo-[5,1-b]quinazolin-9(4H)-one shows a 100% inhibition of allergic response when administered intravenously to the rat at a dose of 0.1 mg/kg; 4,9-dihydro-7-methoxy-4-methyl-9-oxo-pyrazolo[5,1-b]quinazoline-2-carboxylic acid shows a 57% inhibition of the allergic response when administered intravenously to the rat at a dose of 0.1 mg/kg; 4,9-dihydro-4-methyl-9-oxopyrazolo-[5,1-b]quinazoline-2-carboxylic acid shows a 100% inhibition of the allergic response when administered intravenously to the rat at a dose of 0.5 mg/kg; 4,9-dihydro-6,7-dimethoxy-4-methyl-9-oxopyrazolo[5,1-b]quinazoline-2-carboxylic acid shows a 100% inhibition of the allergic response when administered intraperitoneally to the rat at a dose of 5 mg/kg; 4,9-dihydro-5-methoxy-4-methyl-9-oxo-pyrazolo[5,1-b]quinazoline-2-carboxylic acid shows a 60% inhibition of the allergic response when administered intraperitoneally to the rat at a dose of 5 mg/kg; 4,9-dihydro-9-oxo-5-propoxy-4-propyl-pyrazolo[5,1-b]quinazoline-2-carboxylic acid shows a 72% inhibition of the allergic response when administered intraperitoneally to the rat at a dose of 5 mg/kg.

The compositions of the invention can be administered in a variety of dosage forms such as tablets or capsules and liquids for oral or parenteral use. The dosage forms may contain, in addition to the active component, any of the usual compounding excipients such as flavors, colors, stabilizers and tableting materials such as binders, fillers and lubricants. The dosage requirements may vary with the particular composition being employed and may depend on the severity of the symptoms being presented and the size of the mammal being treated. In general, an amount of from about 0.1 to about 10 mg/kg of the active component in single or divided doses will be sufficient to accomplish the method of the invention.

The invention is illustrated by the following examples, in which all temperatures are expressed in degrees Celsius, as is also the case for the preparative examples.

## Example 1

4,9-Dihydro-5-methoxy-9-oxo-pyrazolo[5,1-b]quinazoline-2-carboxylic acid.

A mixture of 2-amino-3-methoxybenzoic acid hydrazide (23,6 g; 0.13 mole) and 90% diethyl oxalacetate, sodium salt (32.7 g; 0.14 mole) in water (400 ml) is heated under reflux for 1.5 hrs. To the resulting yellow solution is added sodium carbonate (14.8 g; 0.14 mole) and the solution is refluxed for an additional hour. The cooled reaction mixture is cautiously treated with conc. HCl (0.28 mole) and the precipitated solid is filtered off, washed with water and recrystallized from methanol (1500 ml). Yield 5.8 g; mp 271—272°C. (d).

## Example 2

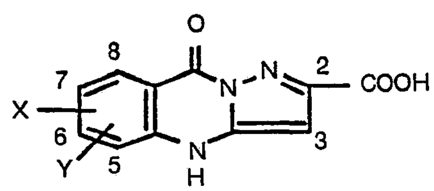

The compounds below are prepared from appropriately substituted anthranilic acid hydrazides by the procedure of Example 1.

| | X | Y | mp | SOLVENT FOR RECRYSTALLIZATION |
|---|---|---|---|---|
| a) | H | H | 315—320°C (d) | DMF |
| b) | 7—Cl | H | 356—359°C (d) | DMF |
| c) | 7—$OCH_3$ | H | 310—320°C | DMF |
| d) | 5—$CH_3$ | H | 268—274°C | MeOH—$H_2O$ |
| e) | 7—$CH_3$ | H | 290—295°C | DMF—$Et_2O$ |
| f) | 6—$OCH_3$ | 7—$OCH_3$ | 305—308°C (d) | DMF |
| g) | 7—F | H | 300°C | DMF |
| h) | 8—$CF_3$ | H | 298—302°C | DMF—$CH_3OH$ |
| i) | 8—$OCH_3$ | H | 293—295°C | DMF |

Example 3

4,9-Dihydro-7-hydroxy-9-oxo-pyrazolo[5,1-b]quinazoline-2-carboxylic acid.

A suspension of 4,9-dihydro-7-methoxy-9-oxo-pyrazolo[5,1-b]quinazoline-2-carboxylic acid (2.0 g; 0.0077 mole) in 48% hydrobromic acid (30 ml) and glacial acetic acid (50 ml) is refluxed for 23 hrs. The mixture is cooled and then diluted with water (25 ml). The product, which precipitates out, is collected by filtration and recrystallized from DMF-Ether (1:1, 90 ml). Yield 1.1 g; mp 285—286°C. (d).

Example 4

4,9-Dihydro-5-hydroxy-9-oxo-pyrazolo[5,1-b]quinazoline-2-carboxylic acid.

From 4,9-dihydro-5-methoxy-9-oxo-pyrazolo[5,1-b]quinazoline-2-carboxylic acid (1.04 g) 48% hydrobromic acid (25 ml) and acetic acid (25 ml), following the procedure of Example 3, there is obtained the desired product (0.75 g) as the quarter hydrate. mp 326—330°C.

Example 5

4,9-Dihydro-7-methylthio-9-oxo-pyrazolo[5,1-b]quinazoline-2-carboxylic acid.

A mixture of 2-amino-5-methylthiobenzoic acid hydrazide (2.5 g; 12.7 mmole) and 90% diethyloxalacetate, sodium salt (3.27 g; 14 mmole) in water (50 ml) is heated under reflux for 2 hrs. Sodium carbonate (1.5 g; 14 mmole) solution in water (15 ml) is added to the reaction mixture and the heating is continued for another hour. The reaction mixture is cooled, carefully acidified with conc. HCl (4 ml) and the resulting yellow precipitate is filtered, washed and dried. The product is recrystallized from methanol-water, yield 1.0 g; mp 285—9° (d).

Example 6

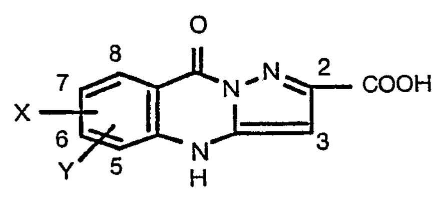

The compounds below are prepared from appropriately substituted anthranilic acid hydrazides by the procedure of Example 5.

| X | Y | mp | SOLVENT FOR CRYSTALLIZATION |
|---|---|---|---|
| 7—$(CH_3)_2CHS$ | H | 275—6°C (d) | DMF |
| 7—$CH_3(CH_2)_3$— | H | 270—1°C (d) | DMF—MeOH |
| 7—$CH_3O$— | 5—$CH_3O$— | 285—7°C (d)[1] | DMF |
| H | 5– 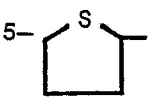 | 253—5°C (d)[2] | MeOH |

[1] Contains 1/3 $CH_3OH$, 1/10 $C_3H_7NO$ as solvent of crystallization.
[2] Contains 1 $CH_3OH$ as solvent of crystallization.

### Example 7

4,9-Dihydro-7-(methylsulfinyl)-9-oxo-pyrazolo[5,1-b]quinazoline-2-carboxylic acid.

A mixture of 4,9-dihydro-7-methylthio-9-oxo-pyrazolo[5,1-b]quinazoline-2-carboxylic acid (5.5 g; 0.02 mole) and 1(N) sodium hydroxide solution (25 ml) in water (500 ml) is chilled to 12° and a solution of sodium metaperiodate (4.28 g, 0.02 mole) in water (150 ml) is added. The reaction mixture is stirred at room temperature for 4 hrs and the resulting solution is cooled and treated with 1(N) HCl (30 ml). The greenish precipitate is filtered, washed and recrystallized from DMF-methanol. Yield 4.4 g; mp 285—90° (d).

### Example 8

4,9-Dihydro-7-(butylsulfinyl)-9-oxo-pyrazolo[5,1-b]quinazoline-2-carboxylic acid, hemihydrate.

From 4,9-dihydro-7-(butylthio)-9-oxo-pyrazolo[5,1-b]quinazoline-2-carboxylic acid (3.17 g, 10 mmole), 1(N) NaOH soln (10 ml) in water (150 ml) and $NaIO_4$ (2.14 g; 10 mmole) in water (50 ml); following the procedure of Example 7, there is obtained 4,9-dihydro-7-butylsulfinyl)-9-oxo-pyrazolo-[5,1-b]quinazoline-2-carboxylic acid, hemihydrate (2.0 g); mp 205—210° (d) remelts at 260°(d) after crystallization from aq. methanol.

### Example 9

4,9-Dihydro-7-(methylsulfonyl)-9-oxo-pyrazolo[5,1-b]quinazoline-2-carboxylic acid.

A mixture of 4,9-dihydro-7-(methylthio)-9-oxo-pyrazolo[5,1-b]quinazoline-2-carboxylic acid (1.1 g; 4 mmole), in glacial acetic acid and 30% $H_2O_2$ (3 ml) is heated under reflux for 1 hr and then stirred at room temperature overnight. The tan solid is filtered off and recrystallized from DMF-ether. Yield 0.95; mp 360—365° (d).

### Example 10

4,9-Dihydro-6,7-dihydroxy-9-oxopyrazolo[5,1-b]quinazoline-2-carboxylic acid, compound with dimethylformamide (1:1).

A mixture of 4,9-dihydro-6,7-dimethoxy-9-oxo-pyrazolo[5,1-b]quinazoline-2-carboxylic acid (1.0 g), 48% HBr(30 ml) and acetic acid (50 ml) is refluxed for 20 hours when the product crystallizes out as yellow solid. The reaction mixture is cooled, diluted with water (25 ml) and the product is filtered. The crude product is recrystallized from DMF (20 ml) and water (200 ml). Yield 350 mg; mp 282—6° (d).

### Example 11

5-Methoxy-2-(1H-tetrazol-5-yl)-pyrazolo[5,1-b]quinazoline-9(4H)-one.

To a warm solution of 4,9-dihydro-5-methoxy-9-oxo-pyrazolo[5,1-b]quinazoline-2-carbonitrile (2.98 g; 0.0124 mole) in DMF (150 ml) is added sodium azide (2.42 g; 0.0372 mole) and ammonium chloride (1.99 g; 0.0372 mole). The reaction mixture is heated at 100°C for 22 hrs, concentrated to 1/3 volume and then poured into ice-water mixture. The mixture is acidified to pH 1.0 with 4 (N) hydrochloric acid (15 ml) and the product filtered off. The crude product is stirred with 0.5 (N) sodium hydroxide solution (600 ml) for 1.0 h, the insoluble material filtered off and the filtrate acidified with conc. HCl (30 ml). The precipitated solid is filtered, washed with water and dried; mp 300°C when recrystallized from DMF. Yield 0.425 g.

By the same procedure starting with the corresponding 6,7-dimethoxy compound, the product obtained in 6,7-dimethoxy2-(1H-tetrazol-5-yl)-pyrazolo[5,1-b]quinazolin-9(4H)-one. Similarly starting with the 7-fluoro and 8-trifluoromethyl compounds, respectively, the products obtained are the 7-fluoro- and 8-trifluoromethyl-2-(1H-tetrazol-5-yl)-pyrazolo[5,1-b]quinazolin-9(4H)-ones.

Example 12

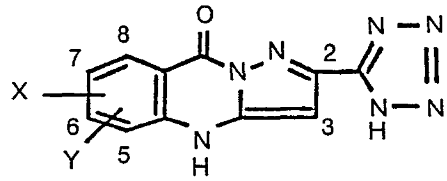

The compounds below are prepared from appropriately substituted 4,9-dihydro-9-oxo-pyrazolo-[5,1-b]quinazoline-2-carbonitriles by the procedure of Example 11.

| X | Y | mp |
|---|---|---|
| H | H | 342—345°C (d) |
| 7—Cl | H | >300°C |
| 7—$OCH_3$ | H | >300°C |

To prepare the sodium salt of the 5-methoxy compound of Example 11, the latter as the free tetrazole is dissolved with warming in an equivalent amount of 0.1N aqueous sodium hydroxide solution, the water is evaporated off and the sodium salt is dried under vacuum. The potassium, calcium and magnesium salts are prepared in the same manner.

Example 13

4-Methyl-2-(1H-tetrazol-5-yl)pyrazolo[5,1-b]-quinazolin-9(4H)-one.

A mixture of 2.25 g of 4,9-dihydro-4-methyl-9-oxo-pyrazolo[5,1-b]quinazoline-2-carbonitrile, 1.9 g of sodium azide, 1.5 g of ammonium chloride and 350 ml of dimethylformamide is stirred and heated at 100°C for 20 hours, then evaporated at reduced pressure. The residue is stirred with 1 liter of diluted hydrochloric acid and the resulting solid is collected by filtration. The solid is stirred with excess saturated aqueous sodium carbonate and the solution is clarified by filtration. The filtrate is acidified with dilute hydrochloric acid and the resulting precipitate of 4-methyl-2-(1H-tetrazol-5-yl)pyrazolo-[5,1-b]quinazolin-9(4H)-one is collected by filtration, washed with water and dried; mp 323°C (dec), after crystallization from dimethylformamide/ethanol.

A sample of the above tetrazole is dissolved in an aqueous solution of one equivalent of sodium hydroxide. The solution is clarified by filtration and freeze-dried to give a residue of the sodium salt. By substituting potassium hydroxide, calcium hydroxide or magnesium hydroxide for sodium hydroxide, one can obtain the potassium, calcium or magnesium salt, respectively.

Example 14

7-Chloro-4-methyl-2-(1H-tetrazol-5-yl)pyrazolo[5,1-b]quinazolin-9(4H)-one.

A mixture of 3.5 g of 7-chloro-4,9-dihydro-4-methyl-9-oxo-pyrazolo[5,1-b]quinazoline-2-carbonitrile, 2.5 g of sodium azide, 2.1 g of ammonium chloride and 600 ml of dimethylformamide is stirred and heated at 100°C for 18 hours, then evaporated at reduced pressure, removing about 575 ml of solvent. The residue is poured into excess dilute hydrochloric acid and the resulting solid is collected by filtration and washed with water. The solid is stirred with excess aqueous sodium carbonate and the solution is clarified by filtration. The filtrate is acidifed with dilute hydrochloric acid and the resulting precipitate of 7-chloro-4-methyl-2-(1H-tetrazol-5-yl)pyrazolo[5,1-b]quinazolin-9(4H)one is collected by filtration, washed with water and dried; mp 312°C (dec), after crystallization from dimethylformamideethanol.

By substituting an equivalent amount of 7-chloro-4-ethyl,4,9-dihydro-9-oxo-pyrazolo[5,1-b]quinazoline-2-carbonitrile for the 7-chloro-4,9-dihydro-4-methyl-9-oxo-pyrazolo[5,1-b]quinazoline-2-carbonitrile in the above example, the product is:

7-Chloro-4-ethyl-2-(1H-tetrazol-5-yl)pyrazolo[5,1-b]quinazolin-9(4H)-one.

Example 15

7-Fluoro-4-methyl-2-(1H-tetrazol-5-yl)pyrazolo[5,1-b]quinazolin-9(4H)-one.

A mixture of 3.0 g of 7-fluoro-4,9-dihydro-4-methyl-9-oxopyrazolo-2-carbonitrile and 500 ml of dimethylformamide is stirred and heated at 100°C for 2 hours, then treated with 1.9 g of ammonium chloride and 2.3 g of sodium azide, and stirred and heated at 100°C for an additional 18 hours. The mixture is cooled, poured into 2.5 liters of ice water and acidified to pH 2 with concentrated hydrochloric acid. The resulting precipitate of 7-fluoro-4-methyl-2-(1H-tetrazol-5-yl)pyrazolo[5,1-b]-

quinazolin-9(4H)-one is collected by filtration, washed with water and dried; mp 350—360°C, after crystallization from dimethylformamide.

Example 16

7-Methoxy-4-methyl-2-(1H-tetrazol-5-yl)pyrazolo[5,1-b]quinazolin-9(4H)-one.

A mixture of 2.5 g of 4,9-dihydro-7-methoxy-4-methyl-9-oxopyrazolo[5,1-b]quinazoline-2-carbonitrile, 1.9 g of sodium azide, 1.5 g of ammonium chloride and 400 ml of dimethylformamide is stirred and heated at 100°C for 22 hours, then evaporated at reduced pressure. The residue is stirred with 100 ml of tetrahydrofuran and the mixture is diluted with 1 liter of ice water. The resulting solid is collected by filtration, washed with water and dissolved in 300 ml of 0.5 *N* aqueous sodium hydroxide. The solution is clarified by filtration and the filtrate is acidified with concentrated hydrochloric acid. The resulting precipitate of 7-methoxy-4-methyl-2-(1H-tetrazol-5-yl)pyrazolo[5,1-b]quinazolin-9(4H)-one is collected by filtration, washed with water and dried; mp 287—288°C, after crystallization from dimethylformamide/ethanol.

By substituting an equivalent amount of 4,9-dihydro-4,7-dimethyl-9-oxo-pyrazolo[5,1-b]quinazoline-2-carbonitrile for the 4,9-dihydro-7-methoxy-4-methyl-9-oxo-pyrazolo[5,1-b]quinazoline-2-carbonitrile in the above example, the product is:
4,7-Dimethyl-2-(1H-tetrazol-5-yl)pyrazolo[5,1-b]quinazolin-9(4H)-one.

Example 17

METHOD A:

4,9-Dihydro-4-methyl-9-oxo-pyrazolo[5,1-b]quinazoline-2-carboxylic acid, ethyl ester.

A stirred suspension of 5.3 g of a 50% mineral oil dispersion of sodium hydride in 30 ml of dimethylformamide is treated dropwise at −10°C, under a nitrogen atmosphere, with a solution of 15.6 g of 5-oxo-2-pyrazoline-3-carboxylic acid, ethyl ester, in 65 ml of dimethylformamide. After the evolution of hydrogen ceases, the mixture is stirred for 2 hours while allowing the temperature to rise to +10°C. The mixture is recooled to −10°C and treated dropwise, with stirring, with a solution of 21.6 g of 90% N-methylisatoic anhydride in 150 ml of dimethylformamide, then stirred for 18 hours while allowing the temperature to rise to 20—25°C. The mixture is heated at 90°C for 1 hour, cooled and poured into 3 liters of ice water containing 10 ml of concentrated hydrochloric acid (final pH about 5). The resulting precipitate is collected by filtration and the filtrate is extracted several times with 600 ml portions of dichloromethane. The precipitate is dissolved in 1 liter of dichloromethane and the solution is combined with the above dichloromethane extracts. The combined dichloromethane solution is washed with water, dried and evaporated. The residual oil is triturated with methanol and the resulting solid is collected, dissolved in tetrahydrofuran and the solution is decolorized by passage through an activated magnesium silicate colum (Florisil R). The word "Florisil" is a registered Trade Mark. The filtrate is concentrated to a small volume, diluted with ethanol and chilled. The resulting crystalline precipitate of 4,9-dihydro-4-methyl-9-oxo-pyrazolo[5,1-b]quinazoline-2-carboxylic acid, ethyl ester is collected by filtration; mp 239—241°C, after recrystallization from dichloromethane/ethanol.

METHOD B:

4,9-Dihydro-4-methyl-9-oxo-pyrazolo[5,1-b]quinazoline-2-carboxylic acid, methyl ester.

A mixture of 2.29 g of 4,9-dihydro-9-oxo-pyrazolo[5,1-b]quinazoline-2-carboxylic acid, 5.52 g of anhydrous potassium carbonate and 34.2 g of methyl iodide in 100 ml of dimethyl formamide is stirred at room temperature for 3 days. Water is added and the white solid is filtered off to give 2.2 g (86%) of the ester; mp 287—289°C (d).

Example 18

7-Chloro-4,9-dihydro-4-methyl-9-oxo-pyrazolo[5,1-b]quinazoline-2-carboxylic acid, ethyl ester.

By substituting 23.2 g of 5-chloro-N-methylisatoic anhydride for the N-methylisatoic anhydride in Example 17A, there is obtained 7-chloro-4,9-dihydro-4-methyl-9-oxo-pyrazolo[5,1-b]quinazoline-2-carboxylic acid, ethyl ester; mp 272—274°C, after recrystallization from dichloromethane/ethanol.

Example 19

7-Fluoro-4,9-dihydro-4-methyl-9-oxo-pyrazolo[5,1-b]quinazoline-2-carboxylic acid, ethyl ester.

By substituting 21.5 g of 5-fluoro-N-methylisatoic anhydride for the N-methylisatoic anhydride in Example 17A, there is obtained 7-fluoro-4,9-dihydro-4-methyl-9-oxo-pyrazolo[5,1-b]quinazoline-2-carboxylic acid, ethyl ester; mp 288—290°C, after recrystallization from dichloromethane/ethanol.

Example 20

4,9-Dihydro-7-methoxy-4-methyl-9-oxo-pyrazolo[5,1-b]quinazoline-2-carboxylic acid, ethyl ester

By substituting 23.1 g of 5-methoxy-N-methylisatoic anhydride for the N-methylisatoic anhydride in Example 17A, there is obtained 4,9-dihydro-7-methoxy-4-methyl-9-oxo-pyrazolo[5,1-b]quinazoline-2-carboxylic acid, ethyl ester; mp 249—251°C, after recrystallization from dichloromethane/ethanol.

Example 21

4,9-Dihydro-4,7-dimethyl-9-oxo-pyrazolo[5,1-b]quinazoline-2-carboxylic acid, ethyl ester

By substituting 21.0 g of N,5-dimethylisatoic anhydride for the N-methylisatoic anhydride in Example 17A, there is obtained, 4,9-dihydro-4,7-dimethyl-9-oxo-pyrazolo[5,1-b]quinazoline-2-carboxylic acid, ethyl ester, mp 250—252°C, after recrystallization from dichloromethane/ethanol.

Example 22

7-Chloro-4-ethyl-4,9-dihydro-9-oxo-pyrazolo[5,1-b]quinazoline-2-carboxylic acid, ethyl ester

By substituting 31.6 g of 5-chloro-N-ethylisatoic anhydride for the N-methylisatoic anhydride in Example 17A, there is obtained 7-chloro-4-ethyl-4,9-dihydro-9-oxo-pyrazolo[5,1-b]quinazoline-2-carboxylic acid, ethyl ester; mp 256—257°C, after recrystallization from dimethyl formamide/-methanol.

Example 23

4,9-dihydro-5-methoxy-4-methyl-9-oxo-pyrazolo[5,1-b]quinazoline-2-carboxylic acid

A mixture of 4,9-dihydro-5-methoxy-9-oxo-pyrazolo[5,1-b]quinazoline-2-carboxylic acid, hydrate (1:0.25) (2.64 g; 0.01 mole), anhydrous potassium carbonate (5.52 g; 0.04 mole) and methyl iodide (15 ml) in dimethyl formamide (75 ml) is stirred at room temperature for 2 days. Water is added and the grey solid is filtered off and taken up in 2(N) sodium hydroxide solution (50 ml) and methanol (50 ml). The mixture is heated under reflux for 2 hrs and the methanol is distilled off *in vacuo*. The aqueous solution is acidified with conc. HCl and the yellow precipitate is collected and recrystallized from methanol (275 ml), water (5 ml). Yield 0.33 g; mp 255°C (d).

Example 24

4,9-Dihydro-6,7-dimethoxy-4-methyl-9-oxo-pyrazolo[5,1-b]quinazoline-2-carboxylic acid

From 4,9-dihydro-6,7-dimethoxy-9-oxo-pyrazolo[5,1-b]quinazoline-2-carboxylic acid (1.5 g; 5.2 mmole), anhydrous potassium carbonate (3.0 g; 22 mmole), methyl iodide (15 ml) and DMF (50 ml), following the procedure of Example 23, there is obtained 4,9-dihydro-6,7-dimethoxy-4-methyl-9-oxo pyrazolo[5,1-b]quinazoline-2-carboxylic acid, methyl ester (1.8 g); mp 301—303° (d). The crude methyl ester (1.8 g) is hydrolyzed with 1(N) sodium hydroxide solution (50 ml) as in Example 11 to give 4,9-dihydro-6,7-dimethoxy-4-methyl-9-oxo-pyrazolo[5,1-b]quinazoline-2-carboxylic acid (0.95 g) mp 284—5°C (d), after crystallization from DMF/methanol.

Example 25

4,9-dihydro-9-oxo-5-propoxy-4-propyl-pyrazolo[5,1-b]quinazoline-2-carboxylic acid, hydrate (10:1)

A mixture of 4,9-dihydro-5-hydroxy-9-oxo-pyrazolo[5,1-b]quinazoline-2-carboxylic acid, hydrate (1:0.25) (0.74 g; 3 mmole), anhydrous potassium carbonate (2.76 g; 20 mmole) and n-propyl iodide (3 ml) in dimethyl formamide (15 ml) is stirred at room temperature for 4 days. The inorganic solids are filtered off and the filtrate is evaporated to dryness, *in vacuo*. The residue is taken up in 2(N) sodium hydroxide solution (25 ml) and warmed on a steam bath for 2 hrs. The mixture is allowed to stand at room temperature overnight and then is acidified with conc. HCl. The precipitate is filtered, washed and dried. Yield 0.125 g; mp 179—83°C.

Example 26

4,9-Dihydro-4-methyl-9-oxo-pyrazolo[5,1-b]quinazoline-2-carboxylic acid

A mixture of 5.8 g of 4,9-dihydro-4-methyl-9-oxo-pyrazolo[5,1-b]quinazoline-2-carboxylic acid, ethyl ester, 50 ml of 1*N* aqueous sodium hydroxide and 400 ml of methanol is heated at reflux until complete solution is attained (about 30 minutes). The solution is evaporated at reduced pressure to remove methanol; the residue is diluted with water and acidified with 1*N* hydrochloric acid. The resulting precipitate is 4,9-dihydro-4-methyl-9-oxo-pyrazolo[5,1-b]quinazoline-2-carboxylic acid is collected by filtration, washed with water and dried; mp 265°C, after crystallization from dimethylformamide/methanol.

Example 27

7-Chloro-4,9-dihydro-4-methyl-9-oxo-pyrazolo[5,1-b]quinazoline-2-carboxylic acid

From 0.6 g of 7-chloro-4,9-dihydro-4-methyl-9-oxo-pyrazolo[5,1-b]quinazoline-2-carboxylic acid ethyl ester, 22 ml of 0.1*N* aqueous sodium hydroxide and 66 ml of methanol, following the procedure of Example 26, there is obtained 7-chloro-4,9-dihydro-4-methyl-9-oxo-pyrazolo[5,1-b]quinazoline-2-carboxylic acid, mp 280°C, after crystallization from dimethylformamide/methanol.

Example 28

4,9-Dihydro-4,7-dimethyl-9-oxo-pyrazolo[5,1-b]quinazoline-2-carboxylic acid

From 3.5 g of 4,9-dihydro-4,7-dimethyl-9-oxo-pyrazolo[5,1-b]quinazoline-2-carboxylic acid, ethyl ester, 50 ml of 1*N* aqueous sodium hydroxide and 200 ml of methanol, following the procedure of Example 26, there is obtained 4,9-dihydro-4,7-dimethyl-9-oxo-pyrazolo[5,1-b]quinazoline-2-carboxylic

acid; mp 269°C, after crystallization from dimethylformamide/ethanol.

Example 29

4,9-Dihydro-7-methoxy-4-methyl-9-oxo-pyrazolo[5,1-b]quinazoline-2-carboxylic acid

From 6.0 g of 4,9-dihydro-7-methoxy-4-methyl-9-oxo-pyrazolo[5,1-b]quinazoline-2-carboxylic acid, ethyl ester, 100 ml of 1*N* aqueous sodium hydroxide and 200 ml of methanol following the procedure of Example 26, there is obtained 4,9-dihydro-7-methoxy-4-methyl-9-oxo-pyrazolo[5,1-b]quinazoline-2-carboxylic acid; mp 259°C, after crystallization from dimethylformamide/ethanol.

Example 30

7-Fluoro-4,9-dihydro-4-methyl-9-oxo-pyrazolo[5,1-b]quinazoline-2-carboxylic acid

From 3.5 g of 7-fluoro-4,9-dihydro-4-methyl-9-oxo-pyrazolo[5,1-b]quinazoline-2-carboxylic acid, ethyl ester, 50 ml of 1*N* aqueous sodium hydroxide and 175 ml of methanol, following the procedure of Example 26, there is obtained 7-fluoro-4,9-dihydro-4-methyl-9-oxo-pyrazolo[5,1-b]quinazoline-2-carboxylic acid; mp 295°C, after crystallization from dimethylformamide/methanol.

Example 31

7-Chloro-4-ethyl-4,9-dihydro-9-oxo-pyrazolo[5,1-b]quinazoline-2-carboxylic acid

From 11.2 g of 7-chloro-4-ethyl-4,9-dihydro-9-oxo-pyrazolo[5,1-b]quinazoline-2-carboxylic acid, ethyl ester, 100 ml of 1*N* aqueous sodium hydroxide and 250 ml of methanol, following the procedure of Example 26, there is obtained 7-chloro-4-ethyl-4,9-dihydro-9-oxo-pyrazolo[5,1-b]quinazoline-2-carboxylic acid; mp 267—268°C (d), after crystallization from dimethylformamide/methanol.

Example 32

4,9-Dihydro-4-methyl-9-oxo-N-1H-tetrazol-5-yl-pyrazolo[5,1-b]quinazoline-2-carboxamide

To a warm mixture of 4-methyl-4,9-dihydro-9-oxo-pyrazolo[5,1-b]quinazoline-2-carboxylic acid (.19 g; 0.0078 mole) in dimethylformamide (50 ml) is added, 1,1′-carbonyl-diimidazole (2.78 g; 0.0172 mole) and the mixture is heated at 100°C for 15 min. 5-Aminotetrazole monohydrate (0.88 g; 0.0086 mole) is then added to the hot solution and the heating continued for another 2.5 hours. The reaction mixture is cooled to 50°C, the solid is filtered and washed successively with methanol and ether and dried. Yield 2.4 g; mp 338—340°C. The sodium salt is prepared by mixing the product with an equivalent amount of 0.1N aqueous sodium hydroxide solution with warming to cause the product to dissolve, evaporating the water off and drying the sodium salt product in vacuum. The potassium, calcium and magnesium salts are prepared by the same procedure.

Example 33

4,9-Dihydro-5-methoxy-9-oxo-N-1H-tetrazol-5-yl-pyrazolo[5,1-b]quinazoline-2-carboxamide

To a suspension of 1-[4,9-dihydro-5-methoxy-9-oxo-pyrazolo[5,1-b]quinazoline-2-yl-carbonyl]-1H-imidazole in dimethylformamide is added a mixture of 5-aminotetrazole monohydrate (1 eq.) and 1,1′-carbonyl-diimidazole (1.1 eq.) in dimethylformamide and the mixture is stirred at 100°C for 2 hrs when the product slowly crystallizes out; mp 280—285°C (d).

Example 34

4,9-Dihydro-7-methylthio-9-oxo-N-1H-tetrazol-5-yl-pyrazolo[5,1-b]quinazoline-2-carboxamide with 1H-imidazole (1:1), hydrate (4:5)

1,1′-carbonyldiimidazole (1.46 g; 9.0 mmole) is added to a warm (90°C) suspension of 4,9-dihydro-7-methylthio-9-oxo-pyrazolo[5,1-b]quinazoline-2-carboxylic acid (1.1 g; 4 mmole) in DMF (40 ml) 5-amino-1*H* tetrazole monohydrate (0.46 g; 4.5 mmole) is added and the solution is heated at 90°C for 3.5 hrs. The product crystallizes out on cooling. Yield 0.3 g; mp 285°C (d).

Example 35

4,9-Dihydro-y-[(1-methylethyl)thio]-9-oxo-N-1H-tetrazol-5-yl pyrazolo[5,1-b]quinazoline-2-carboxamide with 1H-imidazole (4:1)

From 4,9-dihydro-7-[(1-methylethyl)thio]-9-oxo-pyrazolo[5,1-b]quinazoline-2-carboxylic acid (0.91 g; 3 mmole), 1,1′-carbonyldiimidazole (1.1 g; 6.8 mmole), DMF (20 ml) and 5-amino-1H-tetrazole, monohydrate (0.35 g; 3.4 mmole) following the procedure of Example 34, there is obtained 4,9 - dihydro - 7 - [(1 - methylethyl)thio] - 9 - oxo - *N* - 1*H* - tetrazol - 5 - yl - pyrazolo[5,1-b]quinazoline - 2 - carboxamide with 1*H* - imidazole (4:1) (0.8 g); mp >300°C (d).

Example 36

4,9-Dihydro-7-butylthio-9-oxo-N-1H-tetrazol-5-yl-pyrazolo[5,1-b]quinazoline-2-carboxamide, with 1H-imidazole (3:1)

From 4,9-dihydro-7-butylthio-9-oxo-pyrazolo[5,1-b]quinazoline-2-carboxylic acid (1.9 g; 6 mmole), DMF (25 ml), 1,1′-carbonyl-diimidazole (2.19 g; 13.5 mmole) and 5-amino-1*H*-tetrazole, monohydrate (0.70 g; 6.8 mmole), following the procedure of Example 34, there is obtained 4,9-dihydro-7-butylthio-9-oxo-*N*-1*H*-tetrazol-5-yl-pyrazolo[5,1-b]quinazoline-2-carboxamide, with 1*H*-imidazole (3:1) (0.4 g); mp 285°C (d).

Example 37

4,9-Dihydro-5,7-dimethoxy-9-oxo-N-1H-tetrazol-5-yl-pyrazolo[5,1-b]quinazoline-2-carboxamide

From 4,9-dihydro-5,7-dimethoxy-9-oxo-pyrazolo[5,1-b]quinazoline-2-carboxylic acid (1.54 g; 5 mmole), DMF (300 ml), 1,1′-carbonyldiimidazole (1.8 g; 11 mmole) and 5-amino-1*H*-tetrazole, monohydrate (0.52 g; 5 mmole), following the procedure of Example 34, there is obtained 4,9 - dihydro - 5,7 - dimethoxy - 9 - oxo - *N* - 1*H* - tetrazol - 5 - yl - pyrazolo[5,1 - b]quinazoline - 2 - carboxamide (0.78 g); mp 338—342°C (d).

Example 38

4,9 - Dihydro - 9 - oxo - 5 - (tetrahydro - 2 - thienyl) - N - 1H - tetrazol - 5 - yl - pyrazolo[5,1 - b]quinazoline - 2 - carboxamide

From 4,9 - dihydro - 9 - oxo - 5 - (tetrahydro - 2 - thienyl) - pyrazolo[5,1 - b]quinazoline - 2 - carboxylic acid, compound with methanol (1:1) (0.40 g; 1.3 mmole), DMF (20 ml); 1,1′ carbonyldiimidazole (0.5 g; 3.1 mmole) and 5-amino-1*H*-tetrazole, monohydrate (0.131 g; 1.3 mmole), following the procedure of Example 34, there is obtained 4,9 - dihydro - 9 - oxo - 5 - (tetrahydro - 2 - thienyl) - *N* - 1*H* - tetrazol - 5 - yl - pyrazolo[5,1 - b]quinazoline - 2 - carboxamide (70 mg); mp >230°C (d).

Example 39

4,9-Dihydro-7-(methylsulfinyl)-9-oxo-N-1H-tetrazol-5-yl-pyrazolo[5,1-b]quinazoline-2-carboxamide with pyridine (4:3)

From 4,9 - dihydro - 7 - (methylsulfinyl) - 9 - oxo - pyrazolo[5,1 - b]quinazoline - 2 - carboxylic acid (1.40 g; 5 mmole), DMF (50 ml), 1,1′-carbonyl-diimidazole (2.43 g; 14.9 mmole) and 5-amino-1-*H*-tetrazole, monohydrate (0.62 g; 6 mmole), following the procedure of Example 34, there is obtained 4,9 - dihydro - 7 - (methylsulfinyl) - 9 - oxo - *N* - 1*H* - tetrazol - 5 - yl - pyrazolo[5,1 - b]quinazoline - 2 - carboxamide with pyridine (4:3), mp >350°C; after crystallization from pyridine ether.

Example 40

Following the procedure of Example 32, the following compounds are prepared:

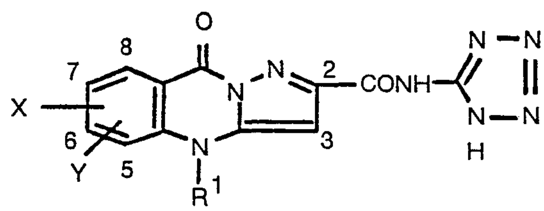

| X | Y | $R^1$ | mp°C | Solvent of Recrystallization or Wash Solvent |
|---|---|---|---|---|
| a) H | H | H | 315—330°(d) | Methanol |
| b) 7-Cl | H | H | >360° | Acetic Acid |
| c) 5-$CH_3O$ | H | H | 280—285°(d) | DMF |
| d) 5-$CH_3$ | H | H | 290—5°(d) | $CH_2Cl_2$ |
| e) 7-OH | H | H | >295°(d) | DMF-MeOH |
| f) 7-$CH_3O$ | H | H | 335°(d) | MeOH-$Et_2O$ |
| g) 8-$CH_3O$ | H | H | 340—4°(d) | DMF |
| h) 6-$CH_3O$ | 7-$CH_3O$ | H | 290—5°(d) | Acetic Acid |

## PREPARATIVE EXAMPLES

### Preparative Example 1

3-Methoxy-2H-3,1-benzoxazine-2,4(1H)dione; (3-methoxyisatoic anhydride)

To a solution of 3-methoxy anthranilic acid (8.36 g; 0.05 mole) is dioxane (75 ml) and benzene (25 ml) is added a 12.5% solution of phosgene in benzene (46 g) with cooling in an ice bath. After the addition, the reaction mixture is stirred at room temperature overnight. The precipitated product is filtered off, washed with benzene and ether, dried and used without further purification. Yield 9.1 g (94%) mp 263—264°C (d).

### Preparative Example 2

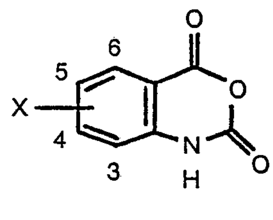

The compounds below are prepared from appropriately substituted anthranilic acids by the procedure of Preparative Example 1.

| X | mp |
|---|---|
| 3-$CH_3$ | 286—288°C (d) |
| 5-$CH_3$ | 245—250°C (d) |
| 5-$OCH_3$ | 244—247°C (d) |
| 6-$OCH_3$ | 260—264°C (d) |

### Preparative Example 3

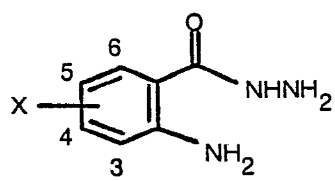

Substituted-2-aminobenzoic acid, hydrazide

The substituted isatoic anhydride (0.14 mole) is slowly added to a cold (+5°C to +10°C) 18% aqueous solution of hydrazine (225 ml). During the exothermic reaction a white solid is formed. After stirring at room temperature overnight, the product is filtered off and washed with water. The hydrazide is used as is or is purified via crystallization before use.

The following compounds are prepared employing the above procedure.

| X | mp | RECRYSTALLIZATION SOLVENT |
|---|---|---|
| 3-$CH_3$ | 155—158°C | — |
| 3-$OCH_3$ | 142—147°C | Benzene |
| 5-$CH_3$ | 137—138°C | — |
| 5-$OCH_3$ | 141—143°C | Water |
| 5-Cl | 133.5—136°C | — |
| 6-$CF_3$ | 120—125°C (d) | — |
| 5-F | 248—251°C | $CH_2Cl_2$-MeOH |
| 6-$OCH_3$ | 151—155°C | — |

Preparative Example 4

4,9-Dihydro-9-oxo-pyrazolo[5,1-b]quinazoline-2-carboxamide

A mixture of 4,9-dihydro-9-oxo-pyrazolo[5,1-b]quinazoline-2-carboxylic acid (5 g; 0.022 mole), thionyl chloride (250 ml) and four drops of pyridine is stirred at room temperature for 24 hrs. The reaction mixture is evaporated to dryness under reduced pressure in a water bath at 30—40°C. The residue is treated with cold (0°C) concentrated ammonium hydroxide solution (200 ml) and allowed to come to room temperature. The product is filtered off, washed with ether and used in the next step without further purification. Yield: 3.2 g; mp 315—325°C. An analytical sample, recrystallized from dimethylformamide, melts at 335—340°C. By the same procedure, starting with the corresponding 7-fluoro compound, the product obtained is 4,9-dihydro-7-fluoro-9-oxo-pyrazolo[5,1-b]quinazoline-2-carboxamide.

Preparative Example 5

4,9-Dihydro-5-methoxy-9-oxo-pyrazolo[5,1-b]quinazoline-2-carboxamide

A mixture of 4,9-dihydro-5-methoxy-9-oxo-pyrazolo[5,1-b]quinazoline-2-carboxylic acid (6.6 g; 0.025 mole) and phosphorus oxychloride (100 ml) is stirred at room temperature overnight. The solid is filtered off, washed with ether, dried and treated with cold (0°C) ammonium hydroxide solution (58%; 50 ml). After standing overnight at room temperature the product is filtered off, dried and used in the next step without further purification. Yield 5.06 g; mp 265—270°C. By the same procedure, starting with the corresponding 6,7-dimethoxy compound, the product obtained is 4,9-dihydro-6,7-dimethoxy-9-oxo-pyrazolo[5,1-b]quinazoline-2-carboxamide.

Preparative Example 6

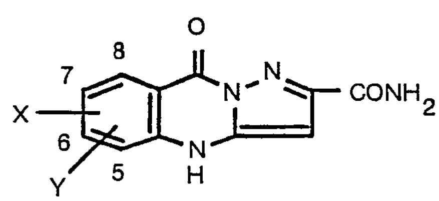

The compounds below are prepared from approximately substituted 4,9-dihydro-9-oxo-pyrazolo[1,5-b]quinazoline-2-carboxylic acids by the procedures of the preceding preparative examples.

| X | Y | mp | METHOD OF PREPARATIVE EXAMPLE |
|---|---|---|---|
| 7-Cl | H | 350—360°C | 4 |
| 7-$OCH_3$ | H | 340—355°C | 5 |

Preparative Example 7

4,9-Dihydro-5-methyl-9-oxo-pyrazolo[5,1-b]quinazoline-2-carboxamide

To a solution of 4,9-dihydro-5-methyl-9-oxo-pyrazolo[5,1-b]quinazoline-2-carboxylic acid (4.86 g; 0.02 mole) in dimethylformamide (50 ml) is added 1,1'-carbonyldiimidazole (4.86 g; 0.03 mole). The reaction mixture is heated at 100°C for 12 min, then cooled and diluted with ether (75 ml) and methylene chloride (25 ml). The tan solid is filtered off and suspended in cold (0°C) DMF (50 ml). Anhydrous ammonia is bubbled through for 10 min and the resulting solution is allowed to stand at room temperature overnight. The dimethylformamide solution is evaporated to dryness under reduced pressure and the residue is washed with methylene chloride and ether and dried. Yield 3.0 g; mp 340—345°C (d). By the same procedure, starting with the corresponding 8-trifluoromethyl compound, the product obtained is 4,9-dihydro-8-trifluoromethyl-9-oxo-pyrazolo[5,1-b]quinazoline-2-carboxamide.

Preparative Example 8

4,9-Dihydro-5-methoxy-9-oxo-pyrazolo[5,1-b]quinazoline-carbonitrile

A suspension of 4,9-dihydro-5-methoxy-9-oxo-pyrazolo[5,1-b]quinazoline-2-carboxamide (5.05 g; 0.0195 mole) in phosphorus oxychloride (100 ml) is heated under reflux for 2 hrs. After standing two days at room temperature, excess phosphorus oxychloride is removed under reduced pressure and the residue suspended in saturated sodium bicarbonate solution (100 ml). The solid is filtered off, washed with water and dried. Yield 3.0 g; mp 292—297°C (d). Starting with the corresponding 7-fluoro, 8-trifluoromethyl and 6,7-dimethoxy compounds, the products obtained are, respectively, the 7-fluoro, 8-trifluoromethyl and 6,7-dimethoxy-4,9-dihydro-9-oxo-pyrazolo[5,1-b]quinazoline-2-carbonitriles.

Preparative Example 9

The compounds below are prepared from appropriately substituted 4,9-dihydro-9-oxo-pyrazolo[5,1-b]quinazoline-2-carboxamides by the procedure of Preparative Example 8.

| X | Y | mp | SOLVENT OF RECRYSTALLIZATION |
|---|---|---|---|
| H | H | 365—375°C (d) | DMF |
| 7-Cl | H | 400—405°C (d) | DMF |
| 7-$OCH_3$ | H | 343—346°C (d) | — |
| 5-$CH_3$ | H | 335—345°C (d) | — |

Preparative Example 10

5-Methylthio-2-nitrobenzoic acid

5-Chloro-2-nitrobenzoic acid (100.8 g; 0.5 mole) is dissolved in water (1.0 l) and 4(N) sodium hydroxide solution (83 ml) (pH 7.5). A solution of $Na_2S.9H_2O$ (132 g; 0.55 mole) in water (300 ml) is added and the mixture is heated at 50—55° for 2.5 hrs. The reaction mixture is then treated with 20% sodium hydroxide solution (100 ml) and dimethyl sulfate (126.2 g; 1.0 mole) and is heated under reflux for 10 hrs. On cooling and acidification with (4 N) HCl (160 ml), the product precipitates out as a yellow solid (101.5 g) which is recrystallized from ether; mp 175—8°C.

Preparative Example 11

Substituted 2-nitrobenzoic acid

The compounds below are prepared from 5-chloro-2-nitrobenzoic acid, sodium sulfide and appropriate alkyl halides ($R^2X$) by the procedure of Preparative Example 10.

| $R^2$ | $R^2X$ | mp |
|---|---|---|
| $(CH_3)CH—$ | $(CH)_2CHBr$ | 133—9° |
| $CH_3(CH_2)_3—$ | n-$C_4H_9Br$ | 105—110° |

Preparative Example 12

Methyl 2-amino-3,5-dimethoxybenzoate

A mixture of methyl 3,5-dimethoxy-2-nitrobenzoate (DE—C 501,609, April 2, 1927; CA. *24*, 47929) (39.0 g; 0.162 mole), 5% Pd on charcoal (2.0 g), methanol (200 ml) and tetrahydrofuran (200 ml) is shaken in an atmosphere of hydrogen at 52 lb pressure (358.5 kilo Pascals) for 46 h when

theoretical amount of hydrogen is absorbed. The catalyst is filtered off and the filtrate is evaporated to dryness. The residue is recrystallized from methanol. Yield 28.6 g; mp 93—5°.

Preparative Example 13

6-Methylthio-2H-3,1-benzoxazine-2,4-(1H)-dione

5-Methylthio-2-nitrobenzoic acid (53.25 g; 0.25 mole) is added to a solution of stannous chloride (225.6 g; 1.0 mole) in conc. HCl (340 ml) and the reaction mixture is brought to 110° and then cooled and concentrated. The concentrate is brought to pH 13 cautiously with 4(N) NaOH. The mixture is then filtered through "Supercel" (registered Trade Mark) and the pH of the filtrate is adjusted to 6.7 and refiltered. The filtrate is cooled and is then treated with 12.5% phosgene in benzene solution. (400 ml). The precipitated solid is filtered, washed and dried. Yield 30.6 g; mp 216—8°.

Preparative Example 14

6-(isopropylthio)-2H-3,1-benzoxazine-2,4-(1H)-dione

From 5-isopropylthio-2-nitrobenzoic acid (12.05 g), following the procedure of preparative example 13, there is obtained 5-isopropylthioisatoic anhydride (7.45 g), mp 219—221°C (d).

Preparative Example 15

6-n-butylthio-2H-3,1-benzoxazine-2,4-(1H)-dione

A solution of phosgene (12.5%) in benzene (40 g; 0.05 mole) is added to a cooled (5°) solution of 5-*n*-butylthio-anthranilic acid (8.9 g; 0.0395 mole) in dioxane (150 ml) and benzene (80 ml). The mixture is stirred at room temperature overnight and the pale green crystals are filtered off and dried. Yield 6.85 g; mp 196—200°C.

Preparative Example 16

6,8-Dimethoxy-2H-3,1-benzoxazine-2,4-(1H)-dione

A mixture of methyl 3,5-dimethoxy anthranilate (21.2 g; 0.1 mole) and 1(N) sodium hydroxide solution (100 ml) is refluxed for 2.0 hrs, cooled and is buffered with dry ice. The solution is treated with 12.5% phosgene in benzene (110 ml) in an ice bath. The mixture is stirred for 4.0 hrs, filtered off the product and dried *in vacuo*. Yield 22.0 g; mp 263—5°C (d).

Preparative Example 17

8-(Tetrahydro-2-thienyl)-2H-3,1-benzoxazine-2,4-(1H)-dione

From methyl 2-amino-3-(tetrahydro-2-thienyl)benzoate (10 g; 0.042 mole), following the procedure of preparative example 16, there is obtained 8-(tetrahydro-2-thienyl)-2H-3,1-benzoxazine-2,4-(1H)-dione (6.0 g), mp 195—9°C (d).

Preparative Example 18

5-Methylthio-2-aminobenzoic acid hydrazide

6-methylthio-2H-3,1-benzoxazine-2,4-(1H)-dione (25.11 g; 0.72 mole) is added to a cold solution of 54.4% hydrazine (75 ml) in water (75 ml). After stirring at room temperature overnight, the white solid is filtered, washed with cold water and dried. Yield 21.3 g; mp 124—127°C.

Preparative Example 19

Substituted-2-aminobenzoic acid hydrazide

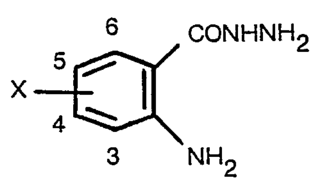

The following compounds are prepared using appropriately substituted isatoic anhydride and following the procedure of Preparative Example 18.

| X | mp |
|---|---|
| 5-$(CH_3)_2CHS$— | 110—115°C |
| 5-$CH_3(CH_2)_3S$— | 92—95°C |
| 3,5-$(CH_3O)_2$— | 137—141°C |
| 3– 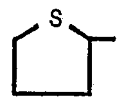 | 123—7°C |

Preparative Example 20

4,9-Dihydro-4-methyl-9-oxo-pyrazolo[5,1-b]quinazoline-2-carboxamide

A mixture of 7.3 g of 4,9-dihydro-4-methyl-9-oxo-pyrazolo[5,1-b]quinazoline-2-carboxylic acid and 250 ml of thionyl chloride is stirred and heated at reflux for 3 hours, then evaporated at reduced pressure. The residue, containing the acid chloride, is stirred with 250 ml of 58% aqueous ammonium hydroxide, then allowed to stand at 20—25°C for 2 hrs. The resulting precipitate of 4,9-dihydro-4-methyl-9-oxo-pyrazolo[5,1-b]quinazoline-2-carboxamide is collected by filtration, washed with water and dried; mp 327—330°C, after crystallization from dimethylformamide/ethanol.

Preparative Example 21

7-Chloro-4,9-dihydro-4-methyl-9-oxo-pyrazolo[5,1-b]quinazoline-2-carboxamide

A mixture of 6.0 g of 7-chloro-4,9-dihydro-4-methyl-9-oxo-pyrazolo[5,1-b]quinazoline-2-carboxylic acid and 200 ml of thionyl chloride is stirred and heated at reflux for 3 hrs, then evaporated at reduced pressure. The residue, containing the acid chloride, is cooled to −10°C, treated with 200 ml of 58% aqueous ammonium hydroxide and stirred at 20—25°C for 3 hrs. The resulting precipitate of 7-chloro-4,9-dihydro-4-methyl-9-oxo-pyrazolo[5,1-b]quinazoline-2-carboxamide is collected by filtration, washed with water and dried; mp >360°C, after crystallization from dimethylformamide/ethanol.

Preparative Example 22

7-Chloro-4-ethyl-4,9-dihydro-9-oxo-pyrazolo[5,1-b]quinazoline-2-carboxamide

By substituting an equivalent amount of 7-chloro-4-ethyl-4,9-dihydro-9-oxo-pyrazolo[5,1-b]quinazoline-2-carboxylic acid for the 7-chloro-4,9-dihydro-4-methyl-9-oxo-pyrazolo[5,1-b]quinazoline-2-carboxylic acid in the procedure of Preparative Example 21, the product is 7-chloro-4-ethyl-4,9-dihydro-9-oxo-pyrazolo[5,1-b]quinazoline-2-carboxamide.

Preparative Example 23

7-Fluoro-4,9-dihydro-4-methyl-9-oxo-pyrazolo[5,1-b]quinazoline-2-carboxamide

A mixture of 10.3 g of 7-fluoro-4,9-dihydro-4-methyl-9-oxo-pyrazolo[5,1-b]quinazoline-2-carboxylic acid and 450 ml of thionyl chloride is stirred and heated at reflux for 3 hours, then evaporated at reduced pressure. The residue, containing the acid chloride, is stirred with 450 ml of 58% aqueous ammonium hydroxide for 2 hours at 20—25°C. The resulting precipitate of 7-fluoro-4,9-dihydro-4-methyl-9-oxo-pyrazolo[5,1-b]quinazoline-2-carboxamide is collected by filtration, washed with water and dried; mp >350°C.

Preparative Example 24

4,9-Dihydro-7-methoxy-4-methyl-9-oxo-pyrazolo[5,1-b]quinazoline-2-carboxamide

A mixture of 2.9 g of 4,9-dihydro-7-methoxy-4-methyl-9-oxo-pyrazolo[5,1-b]quinazoline-2-carboxylic acid and 100 ml of thionyl chloride is stirred and heated at reflux for 1 hour, then evaporated at reduced pressure. The residue, containing the acid chloride, is stirred with 100 ml of 58% aqueous ammonium hydroxide for 2 hrs at 20—25°C. The resulting precipitate of 4,9-dihydro-7-methoxy-4-methyl-9-oxo-pyrazolo[5,1-b]quinazoline-2-carboxamide is collected by filtration washed with water and dried; mp >300°C.

Preparative Example 25

4,9-Dihydro-4,7-dimethyl-9-oxo-pyrazolo[5,1-b]quinazoline-2-carboxamide

By substituting an equivalent amount of 4,9-dihydro-4,7-dimethyl-9-oxo-pyrazolo[5,1-b]quinazoline-2-carboxylic acid for the 4,9-dihydro-7-methoxy-4-methyl-9-oxo-pyrazolo[5,1-b]quinazoline-2-carboxylic acid in Preparative Example 24, there is obtained 4,9-dihydro-4,7-dimethyl-9-oxo-pyrazolo[5,1-b]quinazoline-2-carboxamide.

Preparative Example 26

4,9-Dihydro-4-methyl-9-oxo-pyrazolo[5,1-b]quinazoline-2-carbonitrile

A mixture of 5.3 g of 4,9-dihydro-4-methyl-9-oxo-pyrazolo[5,1-b]quinazoline-2-carboxamide, 5.2 g of thionyl chloride and 65 ml of dimethylformamide is stirred and heated at 57°C for 18 hrs, then evaporated at reduced pressure. The residue is stirred with 600 ml of water and the resulting solid 4,9-dihydro-4-methyl-9-oxo-pyrazolo[5,1-b]quinazoline-2-carbonitrile is collected by filtration, washed with water and dried; mp 307—310°C, after recrystallization from dimethylformamide/ethanol.

Preparative Example 27

7-Chloro-4,9-dihydro-4-methyl-9-oxo-pyrazolo[5,1-b]quinazoline-2-carbonitrile

A mixture of 3.0 g of 7-chloro-4,9-dihydro-4-methyl-9-oxo-pyrazolo[5,1-b]quinazoline-2-carboxamide, 1.6 ml of thionyl chloride and 35 ml of dimethylformamide is stirred and heated at 56°C for 18 hrs, then evaporated at reduced pressure. The residue is stirred with 600 ml of water for 1 hour and the resulting solid 7-chloro-4,9-dihydro-4-methyl-9-oxo-pyrazolo[5,1-b]quinazoline-2-carbonitrile is collected by filtration, washed with water and dried; mp 350°C, after crystallization from dimethylformamide/ethanol.

Preparative Example 28

7-Chloro-4-ethyl-4,9-dihydro-9-oxo-pyrazolo[5,1-b]quinazoline-2-carbonitrile

By substituting an equivalent amount of 7-chloro-4-ethyl-4,9-dihydro-9-oxo-pyrazolo[5,1-b]-quinazoline-2-carboxamide for the 7-chloro-4,9-dihydro-4-methyl-9-oxo-pyrazolo[5,1-b]quinazoline-2-carboxamide in the procedure of Preparative Example 27, the product is 7-chloro-4-ethyl-4,9-dihydro-9-oxo-pyrazolo[5,1-b]quinazoline-2-carbonitrile.

Preparative Example 29

7-Fluoro-4,9-dihydro-4-methyl-9-oxo-pyrazolo[5,1-b]quinazoline-2-carbonitrile

A mixture of 9.2 g of 7-fluoro-4,9-dihydro-4-methyl-9-oxo-pyrazolo[5,1-b]quinazoline-2-carboxamide, 25 ml of thionyl chloride and 175 ml of dimethylformamide is stirred and heated at 65°C for 20 hours, then evaporated at reduced pressure. The residue is stirred with 1 liter of ice water and the resulting solid 7-fluoro-4,9-dihydro-4-methyl-9-oxo-pyrazolo[5,1-b]quinazoline-2-carbonitrile is collected by filtration, washed with water and dried; mp 331—340°C, after crystallization from dimethylformamide/ethanol.

Preparative Example 30

4,9-Dihydro-7-methoxy-4-methyl-9-oxo-pyrazolo[5,1-b]quinazoline-2-carbonitrile

Thionyl chloride (1.5 ml) is added slowly, with stirring to 50 ml of dimethylformamide at 0°C. To this solution is added 2.9 g of 4,9-dihydro-7-methoxy-4-methyl-9-oxo-pyrazolo[5,1-b]quinazoline-2-carboxamide and the mixture is stirred and heated at 50°C for 18 hrs, then evaporated at reduced pressure. The residue is stirred with 600 ml of ice water and the resulting solid 4,9-dihydro-7-methoxy-4-methyl-9-oxo-pyrazolo[5,1-b]quinazoline-2-carbonitrile is collected by filtration, washed with water and dried; mp 316—320°C, after crystallization from dimethylformamide/ethanol.

Preparative Example 31

4,9-Dihydro-4,7-dimethyl-9-oxo-pyrazolo[5,1-b]quinazoline-2-carbonitrile

By substituting an equivalent amount of 4,9-dihydro-4,7-dimethyl-9-oxo-pyrazolo[5,1-b]quinazoline-2-carboxamide for the 4,9-dihydro-7-methoxy-9-oxo-pyrazolo[5,1-b]quinazoline-2-carboxamide in Preparative Example 30, there is obtained 4,9-dihydro-4,7-dimethyl-9-oxo-pyrazolo[5,1-b]-quinazoline-2-carbonitrile.

Preparative Example 32

N,5-Dimethylisatoic anhydride

A solution of 26.6 g of 5-methylisatoic anhydride in 150 ml of dimethylformamide is treated with 17.5 g of anhydrous sodium carbonate, then with 32 g of iodomethane, and the mixture is stirred at 20—25°C for 18 hrs. The reaction mixture is poured into 1.25 liter of ice water and the resulting precipitate of N,5-dimethylisatoic anhydride is collected by filtration, washed with water and dried; mp 166—169°C.

Preparative Example 33

5-Fluoro-N-methylisotoic anhydride

By substituting 27 g of 5-fluoroisatoic anhydride for the 5-methylisatoic anhydride Preparative Example 32, there is obtained 5-fluoro-N-methylisatoic anhydride; mp 150—155°C.

Preparative Example 34

1-(4,9-Dihydro-5-methoxy-9-oxo-pyrazolo[5,1-b]quinazolin -2-yl-carbonyl)-1H-imidazole

To a warm (75°C) solution of 4,9-dihydro-5-methoxy-9-oxo-pyrazolo[5,1-b]quinazoline-2-carboxylic acid (0.52 g) in dimethylformamide (100 ml) is added 1,1'-carbonyl-diimidazole (0.49 g) and the mixture heated at 85°C for 15 min when slowly a yellow solid crystallizes out. Yield 0.24 g; mp 295—300°C.

Preparative Example 35

5-Methylthio-2-aminobenzoic acid

A mixture of 5-methylthio-2-nitrobenzoic acid (25.4 g; 0.119 mole), methanol (200 ml) and Raney nickel (2 g) is shaken in an atmosphere of hydrogen at 50 lb pressure (344.7 kilo Pascals) when theoretical amount of hydrogen is absorbed. The catalyst is filtered off and the filtrate evaporated to dryness. The residue is recrystallized from ether-iso-$Pr_2O$. Yield 7.6 g; mp 145—150°C.

Preparative Example 36

5-n-butylthio-2-aminobenzoic acid

Catalytic hydrogenation of 5-n-butylthio-2-nitrobenzoic acid (10.6 g; 0.042 mole) in methanol (120 ml) in presence of Raney nickel (1 g) by the procedure of Preparative Example 35 gives 5-n-butylthio-2-aminobenzoic acid (8.95 g), mp 69—72°C.

Preparative Example 37

Methyl 2-amino-3-(tetrahydro-2-thienyl)benzoate

A solution of methyl anthranilate (75.5 g; 0.5 mole) in $CH_2Cl_2$ (1.0 l) is cooled to −70°C and a solution of *tert*-butyl hypochlorite (54 g; 0.5 mole) in $CH_2Cl_2$ (150 ml) is added slowly keeping the temperature at −70°C. The resultant N-chloroanthranilate solution is stirred for 1.0 hr and then tetrahydrothiophene (110 ml) is added at such a rate as to maintain the exotherm to less than 10°C. The dark solution is stirred at −70°C for 2.0 hr, triethylamine (125 ml) is added dropwise, and the solution is stirred for 24 hrs. The solvents are removed and the residue is diluted with $CH_2Cl_2$, washed with (1 N) NaOH solution, water and dried. Removal of solvents gives an oil. Unreacted methyl anthranilate is removed under reduced pressure (b.p. 95—100°C/0.35 mm (46.7 Pascals)) at bath temperature at 150°C. The residue is dissolved in $CH_2Cl_2$ and partially chromatographed through silica gel. The solid residue is recrystallized from methanol. Yield 27.0 g; mp 75—9°C.

**Claims**

1. A compound having the following general formula:—

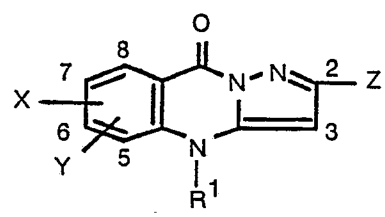

(I)

wherein X is hydrogen, hydroxy, alkyl having from 1 to 6 carbon atoms, alkoxy having from 1 to 6 carbon atoms, halogen, trifluoromethyl or $SO_nR$ wherein R is alkyl having from 1 to 6 carbon atoms and n is 0, 1 or 2; Y is hydrogen, hydroxy, alkyl having from 1 to 6 carbon atoms, alkoxy having from 1 to 6 carbon atoms, or 2-tetrahydrothienyl; $R^1$ is hydrogen or alkyl having from 1 to 6 carbon atoms; Z is COOH,

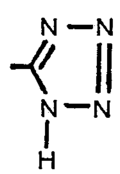

or

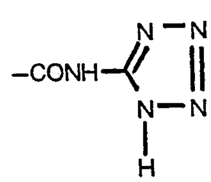

or a pharmaceutically acceptable salt thereof;
provided that when all of X, Y and $R^1$ are hydrogen, Z is not COOH.

2. A compound having formula I as defined in Claim 1, wherein Z is

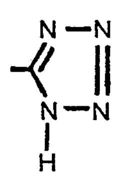

or

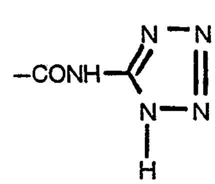

or a pharmaceutically acceptable salt thereof.

3. A compound having the following formula I:

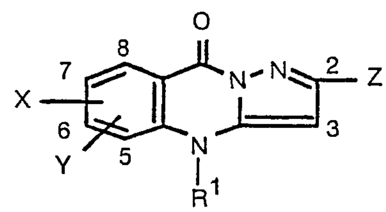

(I)

wherein X is hydrogen, hydroxy, methyl, methoxy, chlorine, fluorine or another halogen, or trifluoromethyl; Y is hydrogen or methoxy; Z is —COOH,

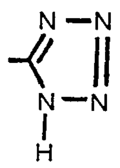

or

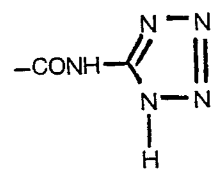

and

$R^1$ is hydrogen, methyl or ethyl; with the proviso that, when $R^1$ is hydrogen and Z is

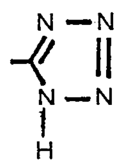

,

X is hydrogen, methyl, methoxy, chlorine, fluorine, trifluoromethyl or hydroxy and Y is hydrogen or, when X is methoxy, is methoxy; or with the proviso that, when $R^1$ is methyl or ethyl and Z is

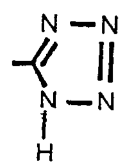

,

Y is hydrogen and X is hydrogen, methyl, methoxy or halogen; or with the proviso that, when Z is

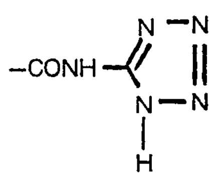

,

$R^1$ is hydrogen or methyl, X is hydrogen, methyl, methoxy, hydroxy, or chlorine, and Y is hydrogen or, when $R^1$ is methoxy, is methoxy or, with the proviso that, when all of X, Y and $R^1$ are hydrogen, Z is not COOH, or a pharmaceutically acceptable salt thereof.

4. A pharmaceutical composition comprising an anti-allergic effective amount of a compound of the formula I:

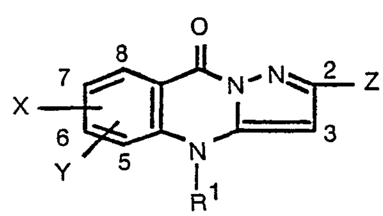

(I)

wherein X is hydrogen, hydroxy, alkyl having from 1 to 6 carbon atoms, alkoxy having from 1 to 6 carbon atoms, halogen, trifluoromethyl, or $SO_nR$ wherein R is alkyl having from 1 to 6 carbon atoms and n is 0, 1 or 2; Y is hydrogen, hydroxy, alkyl having from 1 to 6 carbon atoms, alkoxy having from 1 to 6 carbon atoms, or 2-tetrahydrothienyl; $R^1$ is hydrogen or alkyl having from 1 to 6 carbon atoms; Z is COOH,

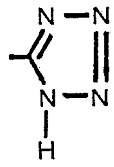 or 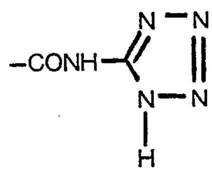

with the proviso that, when all of X, Y and $R^1$ are hydrogen, Z is not COOH, or a pharmaceutically acceptable salt thereof; and a diluent or carrier for that compound or salt thereof.

5. A pharmaceutical composition as claimed in Claim 4, wherein Z is

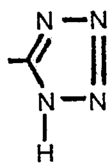 or 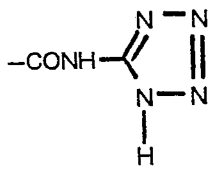 .

6. A pharmaceutical composition as claimed in claim 4, wherein Z is COOH.

7. A pharmaceutical composition comprising a compound as claimed in Claim 3, and a carrier or diluent therefor.

8. A process for preparing a compound of the formula I defined in Claim 1, which process first comprises producing a compound of the following formula:

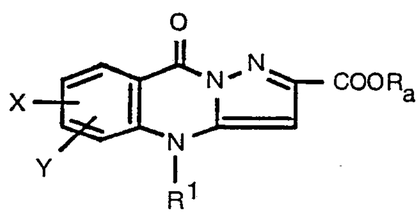

wherein X is hydrogen, hydroxy, alkyl having from 1 to 6 carbon atoms, alkoxy having from 1 to 6 carbon atoms, halogen, trifluoromethyl or $SO_nR$ wherein R is alkyl having from 1 to 6 carbon atoms and n is 0, 1 or 2; Y is hydrogen, hydroxy, alkyl having from 1 to 6 carbon atoms, alkoxy having from 1 to 6 carbon atoms, or 2-tetrahydrothienyl; $R^1$ is alkyl having from 1 to 6 carbon atoms; and $R_a$ is alkyl having from 1 to 6 carbon atoms;
which comprises reacting, in the presence of a strong base, a 3-alkoxycarbonyl-2-pyrazolin-5-one of the formula

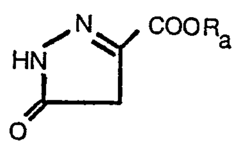

IV

wherein $R_a$ is as defined above,
with a substituted N-alkyl isotoic anhydride of the formula:

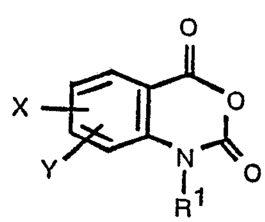

where X, Y and $R^1$ are as defined above;

and then, to produce the compound of formula I in which Z is COOH, hydrolyzing the ester function, $COOR_a$, and isolating the carboxylic acid so produced;

and then to produce a tetrazole compound of formula I in which Z is

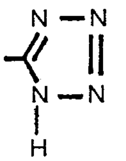
,

converting the carboxylic acid compound to the corresponding acid amide by reaction with an acid halide followed by treatment with ammonia, or directly converting the ester to that acid amide, dehydrating the amide to produce the corresponding nitrile, which is then treated with sodium azide and ammonium chloride to produce the corresponding tetrazole;

or then, to produce a carboxamido tetrazole of formula I in which Z is

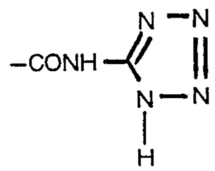
,

converting the carboxylic acid compound to the corresponding acid halide, treating the halide with 5-aminotetrazole to produce the carboxamido tetrazole; or treating the carboxylic acid with 1,1-carbonyl-diimidazole to produce a compound the formula I of Claim 1 but in which Z is

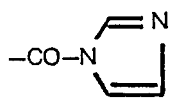

and treating the latter compound with 5-aminotetrazole to produce the desired carboxamidotetrazole.

9. A process for preparing a compound of the formula I defined in Claim 1, which process first comprises producing a compound of the following formula:

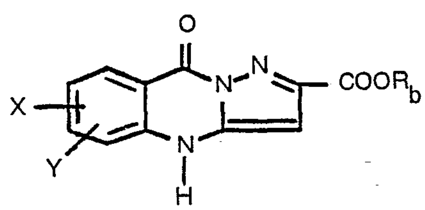

wherein X is a hydrogen, hydroxy, alkyl having from 1 to 6 carbon atoms, alkoxy having from 1 to 6 carbon atoms, halogen, trifluoromethyl, or $SO_nR$ wherein R is alkyl having from 1 to 6 carbon atoms and n is 0, 1 or 2; Y is hydrogen, hydroxy, alkyl having from 1 to 6 carbon atoms, alkoxy having from 1 to 6 carbon atoms, or 2-tetrahydrothienyl; and $R_b$ is alkyl having from 1 to 6 carbon atoms; which comprises reacting a 2-aminobenzioc acid hydrazide of the formula

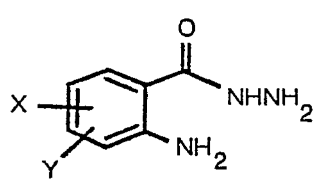

with a dialkyl oxalacetate;

and then to produce the compound of formula I in which Z is COOH, hydrolyzing the ester function, $COOR_b$, and isolating the carboxylic acid so produced, provided that both of X and Y are not hydrogen;

and then to produce a tetrazole compound of formula I in which Z is

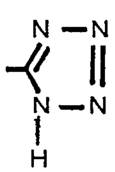
,

converting the carboxylic acid compound to the corresponding acid amide by reaction with an acid halide followed by treatment with ammonia, or directly converting the ester to that acid amide, dehydrating the amide to produce the corresponding nitrile, which is then treated with sodium azide and ammonium chloride to produce the corresponding tetrazole;

or then, to produce a carboxamido tetrazole of formula I in which Z is

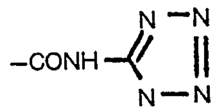

,

converting the carboxylic acid compound to the corresponding acid halide, treating the halide with 5-aminotetrazole to produce the carboxamido tetrazole; or treating the carboxylic acid with 1,1-carbonyldiimidazole to produce a compound the formula I of Claim 1 but in which Z is

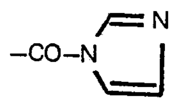

and treating the latter compound with 5-aminotetrazole to produce the desired carboxamidotetrazole.

**Patentansprüche**

1. Verbindung der allgemeinen Formel:

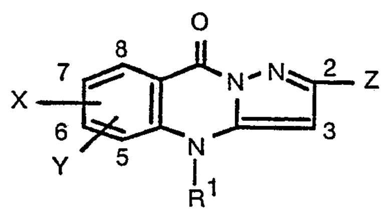

(I)

worin bedeuten:
X ein Wasserstoffatom, eine Hydroxygruppe, eine Alkylgruppe mit 1 bis 6 Kohlenstoffatom(en), eine Alkoxygruppe mit 1 bis 6 Kohlenstoffatom(en); ein Halogenatom, eine Trifluormethylgruppe oder eine Gruppe der Formel $SO_nR$ mit R gleich einer Alkylgruppe mit 1 bis 6 Kohlenstoffatom(en) und $n = 0$, 1 oder 2;
Y ein Wasserstoffatom, eine Hydroxygruppe, eine Alkylgruppe mit 1 bis 6 Kohlenstoffatom(en), eine Alkoxygruppe mit 1 bis 6 Kohlenstoffatom(en) oder eine 2-Tetrahydrothienylgruppe;
$R^1$ ein Wasserstoffatom oder eine eine Alkylgruppe mit 1 bis 6 Kohlenstoffatom(en) und
Z eine Gruppe der Formeln —COOH,

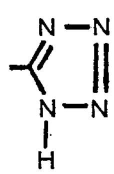

oder

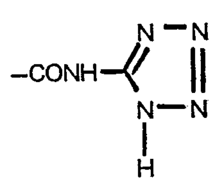

wobei gilt, daß im Falle, daß sämtliche Reste X, Y und $R^1$ für Wasserstoffatome stehen, Z nicht für eine Gruppe der Formel —COOH stehen darf, oder ein pharmazeutisch akzeptables Salz derselben.

2. Verbindung entsprechend Formel (I) nach Anspruch 1, worin Z für eine Gruppe der Formeln

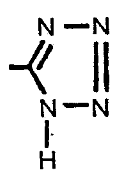

oder

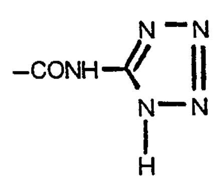

steht, oder ein pharmazeutisch akzeptables Salz derselben.

3. Verbindung der Formel:

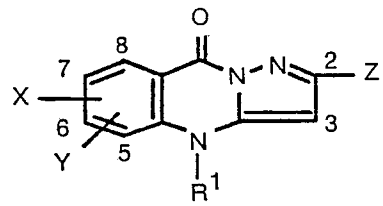

(I)

worin bedeuten:
X ein Wasserstoffatom, eine Hydroxygruppe, eine Methylgruppe, eine Methoxygruppe, ein Chloratom, ein Fluoratom oder ein sonstiges Halogenatom oder eine Trifluormethylgruppe;
Y ein Wasserstoffatom oder eine Methoxygruppe;
Z eine Gruppe der Formeln —COOH,

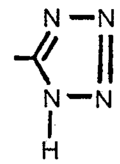

oder

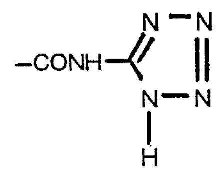

und
$R^1$ ein Wasserstoffatom, eine Methylgruppe oder eine Ethylgruppe, wobei gilt, daß im Falle, daß $R^1$ für ein Wasserstoffatom steht und Z eine Gruppe der Formel

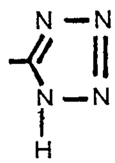

,

darstellt, X einem Wasserstoffatom, einer Methylgruppe, einer Methoxygruppe, einem Chlor- oder Fluoratom, einer Trifluormethylgruppe oder einer Hydroxygruppe entspricht und Y ein Wasserstoffatom bedeutet oder Y, im Falle, daß X eine Methoxygruppe darstellt, ebenfalls eine Methoxygruppe bedeutet, oder ferner, daß im Falle, daß $R^1$ für eine Methyl- oder Ethylgruppe steht und Z eine Gruppe der Formel

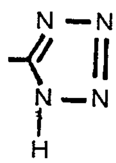

,

darstellt, Y einem Wasserstoffatom entspricht und X ein Wasserstoffatom, eine Methyl- oder Methoxygruppe oder ein Halogenatom bedeutet, oder ferner im Falle, daß Z für eine Gruppe der Formel

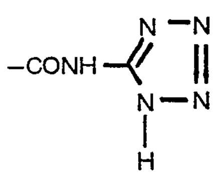

,

steht, $R^1$ einem Wasserstoffatom oder einer Methylgruppe entspricht, X ein Wasserstoffatom, eine Methyl-, Methoxy- oder Hydroxygruppe oder ein Chloratom darstellt und Y für ein Wasserstoffatom steht, oder Y, im Falle, daß $R^1$ eine Methoxygruppe darstellt, ebenfalls eine Methoxygruppe bedeutet, und wobei ferner gilt, daß im Falle, daß sämtliche Reste X, Y und $R^1$ für Wasserstoffatome stehen, Z nicht für eine Gruppe der Formel —COOH stehen darf, oder ein pharmazeutisch akzeptables Salz derselben.

4. Arzneimittel mit einer antiallergisch wirksamen Menge einer Verbindung der Formel:

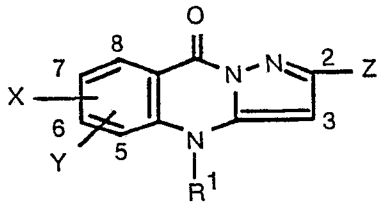

(I)

worin bedeuten:
X ein Wasserstoffatom, eine Hydroxygruppe, eine Alkylgruppe mit 1 bis 6 Kohlenstoffatom(en), eine Alkoxygruppe mit 1 bis 6 Kohlenstoffatom(en); ein Halogenatom, eine Trifluormethylgruppe oder eine Gruppe der Formel $SO_nR$ mit R gleich einer Alkylgruppe mit 1 bis 6 Kohlenstoffatom(en) und $n = 0$, 1 oder 2;
Y ein Wasserstoffatom, eine Hydroxygruppe, eine Alkylgruppe mit 1 bis 6 Kohlenstoffatom(en), eine Alkoxygruppe mit 1 bis 6 Kohlenstoffatom(en) oder eine 2-Tetrahydrothienylgruppe;
$R^1$ ein Wasserstoffatom oder eine Alkylgruppe mit 1 bis 6 Kohlenstoffatom(en) und
Z eine Gruppe der Formeln —COOH,

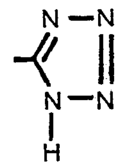

oder

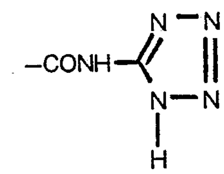

wobei gilt, daß im Falle, daß sämtliche Reste X, Y und $R^1$ für Wasserstoffatome stehen, Z nicht für eine Gruppe der Formel —COOH stehen darf, oder eines pharmazeutisch akzeptablen Salzes derselben und einem Verdünnungsmittel oder Träger für die betreffende Verbindung oder deren Salz.

5. Arzneimittel nach Anspruch 4 (mit einer Verbindung der Formel (I)), in welcher Z für eine Gruppe der Formeln

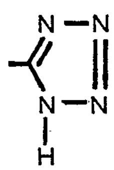

oder

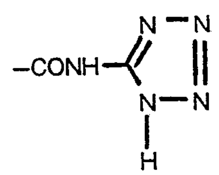

steht.

6. Arzneimittel nach Anspruch 4 (mit einer Verbindung der Formel (I)), in welcher Z für eine Gruppe der Formel —COOH steht.

7. Arzneimittel mit einer Verbindung nach Anspruch 3 und einem Träger oder Verdünnungsmittel hierfür.

8. Verfahren zur Herstellung einer Verbindung der Formel (I) nach Anspruch 1, dadurch gekennzeichnet, daß man zunächst eine Verbindung der Formel:

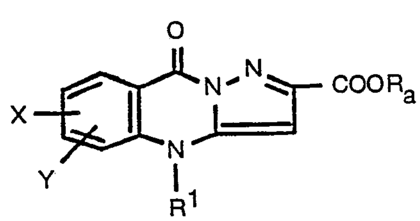

worin bedeuten:
X ein Wasserstoffatom, eine Hydroxygruppe, eine Alkylgruppe mit 1 bis 6 Kohlenstoffatom(en), eine Alkoxygruppe mit 1 bis 6 Kohlenstoffatom(en); ein Halogenatom, eine Trifluormethylgruppe oder eine Gruppe der Formel $SO_nR$ mit R gleich einer Alkylgruppe mit 1 bis 6 Kohlenstoffatom(en) und $n = 0$, 1 oder 2;
Y ein Wasserstoffatom, eine Hydroxygruppe, eine Alkylgruppe mit 1 bis 6 Kohlenstoffatom(en), eine Alkoxygruppe mit 1 bis 6 Kohlenstoffatom(en) oder eine 2-Tetrahydrothienylgruppe;
$R^1$ eine Alkylgruppe mit 1 bis 6 Kohlenstoffatom(en) und
$R_a$ eine Alkylgruppe mit 1 bis 6 Kohlenstoffatom(en), herstellt, indem man in Gegenwart einer starken Base ein 3-Alkoxycarbonyl-2-pyrazolin-5-on der Formel:

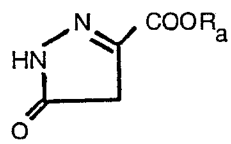

IV

worin $R_a$ die angegebene Bedeutung besitzt, mit einem substituierten N-Alkylisatoesäureanhydrid der Formel:

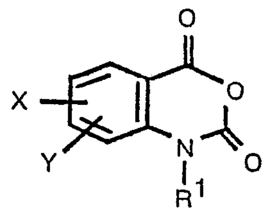

V

worin X, Y und $R^1$ die angegebene Bedeutung besitzen, umsetzt und dann zur Herstellung der Verbindung der Formel (I), worin Z für eine Gruppe der Formel —COOH steht, die Esterfunktion —$COOR_a$ hydrolysiert und die gebildete Carbonsäure isoliert, und anschließend zur Bildung einer Tetrazolverbindung der Formel (I), worin Z für eine Gruppe der Formel

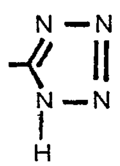

,

steht, die Carbonsäure durch Umsetzung mit einem Säurehalogenid und anschließende Behandlung mit Ammoniak in das entsprechende Säureamid überführt oder den Ester direkt in das Säureamid umwandelt, das Amid zu dem entsprechenden Nitril dehydratisiert und letzteres durch Behandlung mit Natriumazid und Ammoniumchlorid in das entsprechende Tetrazol umwandelt, oder anschließend zur Herstellung eines Carboxyamidotetrazols der Formel (I), worin Z für eine Gruppe der Formel

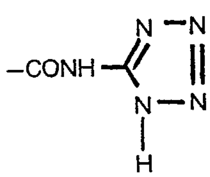

,

steht, die Carbonsäureverbindung in das entsprechende Säurehalogenid überführt, dieses dann mit 5-Aminotetrazol zu dem Carboxamidotetrazol umsetzt oder die Carbonsäure durch Behandeln mit 1,1-Carbonyldiimidazol in eine Verbindung der Formel (I) gemäß Anspruch 1, in welcher jedoch Z für eine Gruppe der Formel

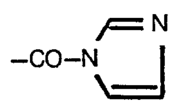

steht, überführt und letztere mit 5-Aminotetrazol zu dem gewünschten Carboxamidotetrazol umsetzt.

9. Verfahren zur Herstellung einer Verbindung der Formel (I) gemäß Anspruch 1, dadurch gekennzeichnet, daß man zunächst eine Verbindung der Formel:

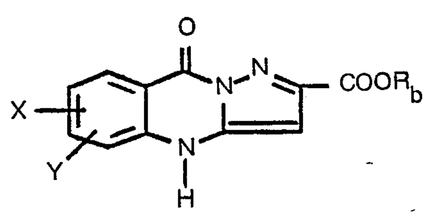

worin bedeuten:

X ein Wasserstoffatom, eine Hydroxygruppe, eine Alkylgruppe mit 1 bis 6 Kohlenstoffatom(en), eine

Alkoxygruppe mit 1 bis 6 Kohlenstoffatom(en); ein Halogenatom, eine Trifluormethylgruppe oder eine Gruppe der Formel $SO_nR$ mit R gleich einer Alkylgruppe mit 1 bis 6 Kohlenstoffatom(en) und $n = 0$, 1 oder 2;
Y ein Wasserstoffatom, eine Hydroxygruppe, eine Alkylgruppe mit 1 bis 6 Kohlenstoffatom(en), eine Alkoxygruppe mit 1 bis 6 Kohlenstoffatom(en) oder eine 2-Tetrahydrothienylgruppe, und
$R_b$ eine Alkylgruppe mit 1 bis 6 Kohlenstoffatom(en) herstellt, indem man ein 2-Aminobenzoesäurehydrazid der Formel:

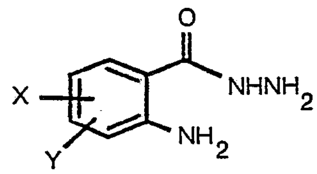

worin nicht beide Reste X und Y gleichzeitig für Wasserstoffatom stehen dürfen, mit einem Dialkyloxalacetat umsetzt und anschließend zur Herstellung einer Verbindung der Formel (I), worin Z eine Gruppe der Formel —COOH darstellt, die Esterfunktion —$COOR_b$ hydrolysiert und die gebildete Carbonsäure isoliert und danach zur Herstellung einer Tetrazolverbindung der Formel (I), worin Z für eine Gruppe der Formel

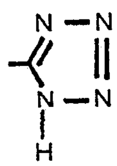 ,

steht, die Carbonsäure durch Umsetzung mit einem Säurehalogenid und anschließende Behandlung mit Ammoniak in das entsprechende Säureamid überführt oder den Ester direkt in das Säureamid umwandelt, das Amid zu dem entsprechenden Nitril dehydratisiert und letzteres durch Behandlung mit Natriumazid und Ammoniumchlorid in das entsprechende Tetrazol umwandelt, oder anschließend zur Herstellung eines Carboxyamidotetrazols der Formel (I), worin Z für eine Gruppe der Formel

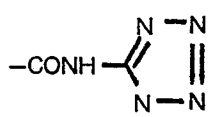 ,

steht, die Carbonsäureverbindung in das entsprechende Säurehalogenid überführt, und dieses dann mit 5-Aminotetrazol in das gewünschte Carboxamidotetrazol überführt oder die Carbonsäure durch Behandeln mit 1,1-Carbonyldiimidazol in einer Verbindung der Formel (I) gemäß Anspruch 1, in welcher jedoch Z für eine Gruppe der Formel

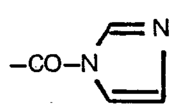

steht, überführt und letztere mit 5-Aminotetrazol zu dem gewünschten Carboxamidotetrazol umsetzt.

**Revendications**

1. Composé répondant à la formule générale suivante:

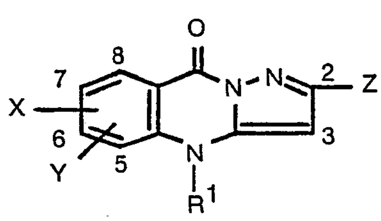 (I)

dans laquelle:
X est un atome d'hydrogène, un groupe hydroxy, alkyle comportant 1 à 6 atomes de carbone, alkoxy

comportant 1 à 6 atomes de carbone, un atome d'halogène, un groupe trifluorométhyle ou un groupe $SO_nR$ dans lequel:

R est un radical alkyle comportant 1 à 6 atomes de carbone; et

n est un nombre égal à 0, 1 ou 2;

Y est un atome d'hydrogène, un groupe hydroxy, un radical alkyle comportant 1 à 6 atomes de carbone, alkoxy comportant 1 à 6 atomes de carbone ou 2-tétrahydrothiényle;

$R^1$ représente un atome d'hydrogène ou un radical alkyle comportant 1 à 6 atomes de carbone;

Z est un groupe COOH, ou un groupe

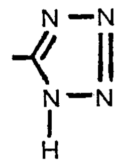

ou

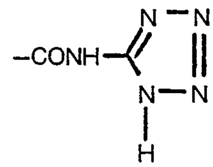

ou bien un de ses sels pharmaceutiquement acceptables, avec cette condition que lorsque tous les groupes X, Y et $R^1$ sont des atomes d'hydrogène, Z n'est pas un groupe COOH.

2. Composé répondant à la formule générale I, selon la revendication 1, dans lequel:

Z est

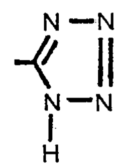

ou

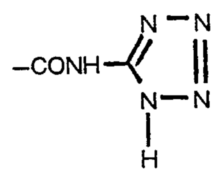

ou un de ses sels pharmaceutiquement acceptables.

3. Composé de formule I,

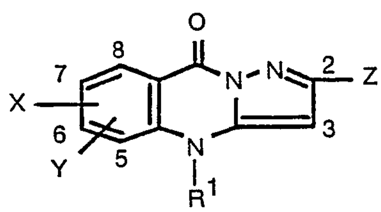

(I)

dans laquelle:

X est un atome d'hydrogène, un radical hydroxy, méthyle, méthoxy, un atome de chlore, de fluor ou un autre halogène ou un groupe trifluorométhyle;

Y est un atome d'hydrogène ou un groupe méthoxy;

Z est un groupe COOH,

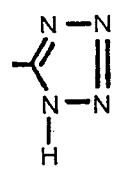

ou

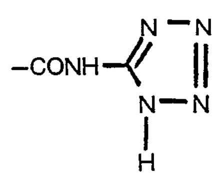

$R^1$ est un atome d'hydrogène, un groupe méthyle ou éthyle; avec cette condition que lorsque $R^1$ est un atome d'hydrogène et Z est

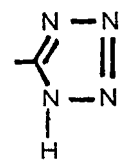

,

X est un atome d'hydrogène, un groupe méthyle, méthoxy, un atome de chlore, de fluor, un groupe trifluorométhyle ou hydroxy et Y est un atome d'hydrogène, ou lorsque X est un groupe méthoxy, y est un groupe méthoxy; ou avec cette condition que lorsque $R^1$ est un groupe méthyle ou éthyle et Z est

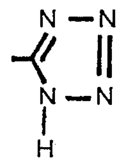
,

Y est un atome d'hydrogène et X est un atome d'hydrogène, un groupe méthyle, méthoxy ou un atome d'halogène; ou avec cette condition que lorsque Z est

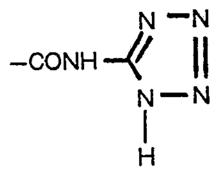
,

$R^1$ est un atome d'hydrogène ou un groupe méthyle, X est un atome d'hydrogène, un groupe méthyle, méthoxy, hydroxy ou un atome de chlore et Y est un atome d'hydrogène ou lorsque $R^1$ est un groupe méthoxy, est un groupe méthoxy avec cette condition que lorsque tous les groupes X, Y et $R^1$ sont des hydrogènes, X n'est pas COOH;
ou bien un de leurs sels pharmaceutiquement acceptables.

4. Composition pharmaceutique renfermant une quantité anti-allergique efficace d'un composé de formule I:

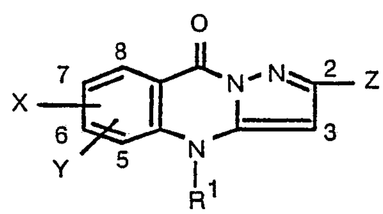
(I)

dans laquelle:
X est un atome d'hydrogène, un groupe hydroxy, un groupe alkyle comportant 1 à 6 atomes de carbone, alkoxy comportant 1 à 6 atomes de carbone, un atome d'halogène, un groupe trifluorométhyle ou un groupe $SO_nR$, dans lequel:
R est un radical alkyle comportant 1 à 6 atomes de carbone; et
n est un nombre égal à 0, 1 ou 2;
Y est un atome d'hydrogène, un groupe hydroxy, un groupe alkyle comportant 1 à 6 atomes de carbone, un groupe alkoxy comportant 1 à 6 atomes de carbone ou 2-tétrahydrothiényle;
$R^1$ est un atome d'hydrogène ou un groupe alkyle comportant 1 à 6 atomes de carbone;
Z est un groupe COOH,

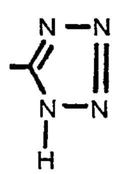
ou
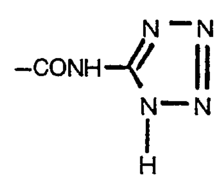

avec cette condition que lorsque tous les groupes X, Y et $R^1$ sont des atomes d'hydrogène, Z n'est pas COOH,
ou un de leurs sels pharmaceutiquement acceptables, et un diluant ou support pour ces composés ou leurs sels.

5. Composition pharmaceutique selon la revendication 4, caractérisé en ce que Z est

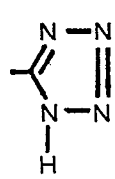
ou
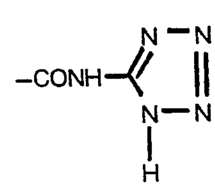

6. Composition pharmaceutique selon la revendication 4, caractérisé en ce que Z est COOH.

7. Composition pharmaceutique renfermant un composé selon la revendication 3, et un support ou diluant.

8. Procédé de préparation d'un composé de formule I selon la revendication 1, ledit procédé comportant d'abord la préparation d'un composé de formule

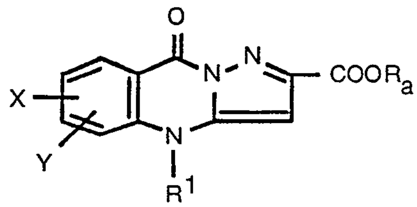

dans laquelle:
X est un atome d'hydrogène, un groupe hydroxy, un groupe alkyle comportant 1 à 6 atomes de carbone, alkoxy comportant 1 à 6 atomes de carbone, un atome d'halogène, un groupe trifluorométhyle ou $SO_nR$ dans laquel:
R est un radical alkyle comportant 1 à 6 atomes de carbone; et
n est un nombre égal à 0, 1 ou 2;
Y est un atome d'hydrogène, un groupe hydroxy, un groupe alkyle comportant 1 à 6 atomes de carbone, un groupe alkoxy comportant 1 à 6 atomes de carbone ou un groupe 2-tétrahydrothiényle;
$R^1$ est un groupe alkyle comportant 1 à 6 atomes de carbone; et
$R_a$ est un groupe alkyle comportant 1 à 6 atomes de carbone;
qui consiste à faire réagir en présence d'une base forte une 3-alkoxycarbonyl-2-pyrazoline-5-one de formule

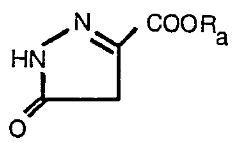

dans laquelle $R_a$ est tel que défini ci-dessus;
avec un anhydride N-alkylisatoïque de formule:

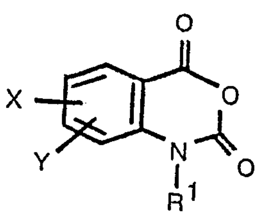

dans laquelle X, Y et $R^1$ sont tels que définis ci-dessus;
puis à préparer le composé de formule I dans lequel Z est COOH, en hydrolisant la fonction ester, $COOR_a$, et en isolant l'acide carboxylique ainsi obtenu;
puis à préparer un dérivé tétrazole de formule I dans lequel Z est:

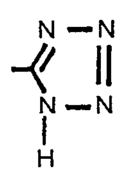 ,

en transformation l'acide carboxylique en l'acide amidé correspondant par réaction avec un halogénure d'acide suivi d'un traitement à l'aide d'ammoniac, ou transformation directe de l'ester en cet acide amidé, déshydratation de l'amide pour donner le nitrile correspondant qui est ensuite traité par un azidure de sodium et le chlorure d'ammonium pour donner le tétrazole correspondant;
ou ensuite pour donner un carboxamido tétrazole de formule I dans lequel
Z est

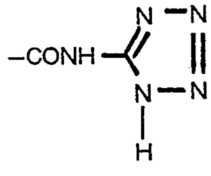 ,

en transformant le dérivé acide carboxylique en l'halogénure d'acide correspondant, traitant l'halogénure par le 5-aminotétrazole pour donner le carboxamidotétrazole;
ou en traitant l'acide carboxylique par le 1,1-carbonyldiimidazole pour donner un composé de formule I selon la revendication 1, mais dans lequel
Z est

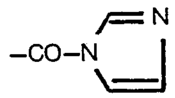

et traitant ce dernier composé par 5-aminotétrazole pour obtanir le carboxamidotétrazole recherché.

9. Procédé de préparation d'un composé de la formule I selon la revendication 1, ce procédé consistant d'abord à préparer un composé de formule suivante:

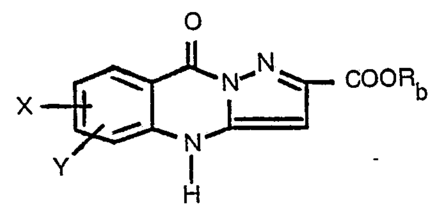

où X est un atome d'hydrogène, un radical hydroxy, un groupe alkyle comportant 1 à 6 atomes de carbone, alkoxy comportant 1 à 6 atomes de carbone, un atome d'halogène, un groupe trifluorométhyle, ou un groupe $SO_nR$ dans lequel:
R est un groupe alkyle comportant 1 à 6 atomes de carbone; et
n est un nombre égal à 0, 1 ou 2;
Y est un atome d'hydrogène, un groupe hydroxy, un groupe alkyle comportant 1 à 6 atomes de carbone, alkoxy comportant 1 à 6 atomes de carbone ou 2-tétrahydrothiényle; et
$R_b$ est un groupe alkyle comportant 1 à 6 atomes de carbone;
procédé selon lequel on fait réagir un hydrazide d'acide 2-aminobenzoïque de formule

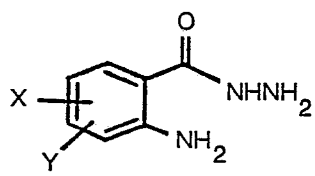

avec l'oxalacétate de dialkyle;
et ensuite on prépare le composé de formule I dans lequel Z est COOH, par hydrolyse de la fonction ester $COOR_b$, et isolement de l'acide carboxylique ainsi obtenu, avec cette condition que X et Y ne sont pas tous les deux de l'hydrogène;
et pour obtenir un tétrazole de formule I dans lequel Z est

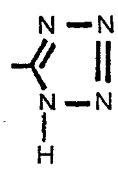 ,

en transformant l'acide carboxylique en l'acide amidé correspondant par réaction avec un halogénure d'acide suivi d'un traitement à l'aide d'ammoniac ou convertissant directement l'ester en cet acide amidé, déshydratation de l'amide pour obtenir le nitrile correspondant qui est ensuite traité par l'azidure de sodium et le chlorure d'ammonium pour donner le tétrazole correspondant; ou ensuite, pour donner un carboxamidotétrazole de formule I dans lequel Z est

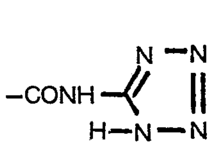 ,

en transformant le dérivé acide carboxylique en l'halogénure d'acide correspondant, traitant l'halogénure par le 5-amidotétrazole pour donner le carboxamidotétrazole;

ou bien en traitant l'acide carboxylique par le 1,1-carbonyldiimidazole pour donner un dérivé de formule I selon la revendication 1, mais dans lequel Z est

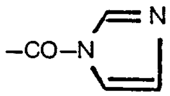

et traitant ce dernier par le 5-aminotétrazole pour donner le carboxamido tétrazole recherché.